# EUROPEAN PATENT SPECIFICATION

(11) **EP 3 166 527 B1**
(45) Date of publication and mention of the grant of the patent: **05.08.2020**
(21) Application number: 15803572.5
(22) Date of filing: 05.06.2015
(51) Int. Cl.: A61B 34/30, A61B 17/94, A61B 90/50, A61B 17/34

(54) **ARTICULATING ROBOTIC PROBES AND SYSTEMS**
ROBOTERSONDEN UND -SYSTEME MIT GELENKEN
SONDES ROBOTIQUES ARTICULÉES ET SYSTÈMES ASSOCIÉS

(30) Priority: 05.06.2014 US 201462008453 P; 20.04.2015 US 201562150223 P
(43) Date of publication of application: 17.05.2017
(73) Proprietor: Medrobotics Corporation, Raynham, MA 02767 (US)
(72) Inventor: ZUBIATE, Brett, Duxbury, MA 02332 (US); NUNES, Nancy, A., Plainville, MA 02762 (US); DARISSE, Ian, J., Southborough, Massachusetts 01772 (US); MORDENTE, Zackery, Raynham, MA 02767 (US); CASTRO, Michael, Raynham, MA 02767 (US); JOHNSTON, Gabriel, Raynham, MA 02767 (US); CALEF, Thomas, Bridgewater, MA 02324 (US); TULLY, Stephen, Quincy, MA 02170 (US); DALEY, Eric, Franklin, MA 02038 (US); COHEN, David, Brookline, MA 02445 (US); STAND, Joseph, A., Holden, MA 02052 (US); ANDERSON, Bob, Norwell, MA 02061 (US); KENNEDY, Kevin, Quincy, MA 02170 (US); FLAHERTY, R., Maxwell, Auburndale, FL 33823 (US); FLAHERTY, J., Christopher, Auburndale, FL 33823 (US)
(74) Representative: Bittner, Bernhard
(86) International application number: PCT/US2015/034424
(87) International publication number: WO 2015/188071

(56) References cited:
- WO-A1-2006/091494
- WO-A1-2013/096610
- WO-A1-2013/096610
- DE-A1- 2 150 595
- US-A- 3 060 972
- US-A- 5 820 623
- US-A1- 2005 195 166
- US-A1- 2009 171 151
- US-A1- 2009 248 038
- US-A1- 2014 012 288
- US-B2- 8 292 807

## Description

### TECHNICAL FIELD

The present inventive concepts generally relate to the field of surgical instruments, and more particularly, to articulating probe assemblies and systems incorporating the same, and systems for performing a surgical procedure.

### BACKGROUND

As less invasive medical techniques and procedures become more widespread, medical professionals such as surgeons may require articulating surgical tools, such as endoscopes, to perform such less invasive medical techniques and procedures that access interior regions of the body via a body orifice such as the mouth. A device according to the preamble of claim 1 is known from WO2013096610.

### SUMMARY

The invention is defined in the appended claims. In an aspect, an articulating probe system comprises: an articulating probe constructed and arranged to articulate in at least one degree of motion and to transition between a flexible state and a rigid state; a feeder assembly in communication with the articulating probe to apply forces on the articulating probe to cause the probe to articulate and to transition between the flexible state and rigid state; a feeder cart on a plurality of wheels that allow manual movement of the cart in a horizontal direction; and a feeder support arm that couples the feeder assembly to the feeder cart.

In some embodiments, at least one of the plurality of wheels comprises a locking wheel.

In some embodiments, the articulating arm includes first and second segments that pivot relative to one another at a pivot joint and wherein one or more pistons are mounted between the first and second segments to support a weight of an upper one of the first and second segments.

In some embodiments, the plurality of wheels comprise caster wheels.

In another aspect, provided is a method for performing a medical procedure using the system.

In an aspect, an articulating probe system comprises: an articulating probe constructed and arranged to articulate in at least one degree of motion and to transition between a flexible state and a rigid state, the articulating probe comprising a plurality of links between a proximal link and a distal link; the distal link of the articulating probe including a side port constructed and arranged to receive a distal end of an elongate tool; and a tool support in communication with the articulating probe for supporting the elongate tool ,the tool support including a tool tube that extends from the tool support at an intermediate portion to the side port of the distal link at a distal portion, the tool tube having a first flexibility in the intermediate portion and having a second flexibility in the distal portion; the second flexibility being greater in flexibility than the first flexibility.

In some embodiments, the tool tube has a circular cross-section and surrounds a side surface of an inserted tool.

In some embodiments, an intermediate link of the articulating probe between the proximal and distal links includes a side port and wherein the tool tube extends through the side port of the intermediate link between the tool support and the side port of the distal link.

In some embodiments, the distal portion of the tool tube includes rib features having a reduced outer diameter.

In some embodiments, the distal portion of the tool tube comprises a material that is different than a material of the intermediate portion.

In some embodiments, the distal portion of the tool tube has a wall thickness that is less than a wall thickness of the intermediate portion.

In another aspect, provided is a method for performing a medical procedure using the system.

In an aspect, an articulating probe system comprises: an articulating probe constructed and arranged to articulate in at least one degree of motion and to transition between a flexible state and a rigid state, the articulating probe comprising a plurality of links between a proximal link and a distal link; the distal link of the articulating probe including a side port constructed and arranged to receive a distal end of an elongate tool; and a tool support in communication with the articulating probe for supporting the elongate tool, the tool support including a tool tube that extends from the tool support at an intermediate portion to the side port of the distal link at a distal portion, wherein an intermediate link of the articulating probe between the proximal and distal links includes a side port and wherein the tool tube extends through the side port of the intermediate link between the tool support and the side port of the distal link.

In some embodiments, the tool tube having a first flexibility in the intermediate portion and having a second flexibility in the distal portion; the second flexibility being greater in flexibility than the first flexibility.

In some embodiments, the tool tube is circular in cross-section and surrounds a side surface of an inserted tool.

In some embodiments, the tool tube is fixedly attached to the side port of the intermediate link.

In some embodiments, the tool tube slides freely through the side port of the intermediate link.

In another aspect, provided is a method for performing a medical procedure using the system.

In an aspect, an articulating probe system comprises: an articulating probe constructed and arranged to articulate in at least one degree of motion and to transition between a flexible state and a rigid state, the articulating probe comprising a plurality of links between a proximal link and a distal link; the distal link of the articulating probe including a side port constructed and arranged to receive a distal end of an elongate tool; and a probe introducer including a neck and a base, a probe channel through the base and neck through which the articulating probe freely passes, the probe channel having an outlet from the base, a tool support coupled to the base of the probe introducer, in communication with the articulating probe for supporting the elongate tool, the tool support having an outlet from the base, wherein an outlet of the probe channel extends a greater distance in a distal direction than the outlet of the tool support.

In some embodiments, the articulating probe system further comprises a flange about the outlet of the probe channel.

In some embodiments, the flange is integral with the base of the probe introducer.

In some embodiments, the flange is coupled to the base of the probe introducer.

In some embodiments, the tool support includes a tool tube that extends from the tool support at an intermediate portion to the side port of the distal link at a distal portion, wherein an intermediate link of the articulating probe between the proximal and distal links includes a side port and wherein the tool tube extends through the side port of the intermediate link between the tool support and the side port of the distal link.

In another aspect, provided is a method for performing a medical procedure using the system.

In an aspect, an articulating probe system comprises: an articulating probe constructed and arranged to articulate in at least one degree of motion and to transition between a flexible state and a rigid state, the articulating probe comprising a plurality of links between a proximal link and a distal link; the plurality of links including a channel constructed and arranged to receive a distal end of an elongate tool, a portion of the elongate tool positioned in the channel, a distal end of the elongate tool being fixed to a distal link of the plurality of links; a feeder assembly in communication with the articulating probe to apply forces on the articulating probe to cause the probe to articulate and to transition between the flexible state and rigid state; a portion of the elongate tool being fixedly attached at an attachment position to the feeder assembly, a service loop in the elongate tool provided between attachment position and the channel, wherein a length in the service loop of the elongate tool is greater than a length of the articulating probe when positioned in its greatest extent of curvature.

In some embodiments, the tool comprises a camera and wherein the service loop of the elongate tool comprises an electrical wire.

In some embodiments, the tool comprises a camera and wherein the service loop of the elongate tool comprises a fiber optic.

In some embodiments, the feeder assembly comprises a carriage for driving the articulating probe in a distal direction and wherein a length of the service loop is greater than a combined length of: the length of the articulating probe when positioned in its greatest extent of curvature; and a distance of the carriage when in a greatest extent in the distal direction.

In some embodiments, the feeder assembly comprises an energy chain coupled between the feeder assembly and the carriage for protecting elements extending through the links.

In another aspect, provided is a method for performing a medical procedure using the system.

In an aspect, an articulating probe system comprises: an articulating probe constructed and arranged to articulate in at least one degree of motion and to transition between a flexible state and a rigid state, the articulating probe comprising a plurality of links between a proximal link and a distal link, the plurality of links comprising a plurality of inner links and a plurality of outer links; the plurality of links including a channel constructed and arranged to receive a distal end of an elongate tool, a portion of the elongate tool positioned in the channel, a distal end of the elongate tool being fixed to a distal link of the plurality of links; a feeder assembly in communication with the articulating probe to apply forces on the articulating probe to cause the probe to articulate and to transition between the flexible state and rigid state, and to cause one of the plurality of inner links and plurality of outer links to perform a steering and locking operation and the other of the plurality of inner links and plurality of outer links to perform a locking operation; a portion of the elongate tool being fixedly attached at an attachment position to the feeder assembly, a service loop in the elongate tool provided between attachment position and the channel, wherein a length in the service loop of the elongate tool is greater than a length of the one of the plurality of inner links and plurality of outer links during its greatest extent when in the steering operation.

In some embodiments, the tool comprises a camera and wherein the service loop of the elongate tool comprises an electrical wire.

In some embodiments, the tool comprises a camera and wherein the service loop of the elongate tool comprises a fiber optic.

In some embodiments, the feeder assembly comprises a carriage for driving the articulating probe in a distal direction and wherein a length of the service loop is greater than a combined length of: the one of the plurality of inner links and plurality of outer links during its greatest extent when in the steering operation; and a distance of the carriage when in a greatest extent in the distal direction.

In some embodiments, the feeder assembly comprises an energy chain coupled between the feeder assembly and the carriage for protecting elements extending through the links.

In another aspect, provided is a method for performing a medical procedure using the system.

In an aspect, an articulating probe system comprises: an articulating probe constructed and arranged to articulate in at least one degree of motion and to transition between a flexible state and a rigid state, the articulating probe comprising a plurality of links between a proximal link and a distal link; a feeder assembly in communication with the articulating probe to apply forces on the articulating probe to cause the probe to articulate and to transition between the flexible state and rigid state; a plurality of cables in communication with the plurality of links; the feeder assembly further comprising: cable bobbins at which proximal ends of the plurality of cables are wound; motor assemblies, each corresponding to one of the cable bobbins, for driving the cable bobbins, the motor assemblies including motion resistance mechanisms that substantially prevent rotation of the bobbins as a result of forces transferred through the cables, as encountered by the articulating probe.

In some embodiments, the motor assemblies comprise: a motor; a gear assembly; and a capstan in communication with the cable bobbin.

In some embodiments, the gear assembly comprises a worm gear assembly.

In some embodiments, the gear assembly comprises at least one of a ratchet and pawl mechanism or a magnetic position holding assembly.

In some embodiments, the motor comprises one of a stepper motor, a closed-loop servomotor, and a DC motor having a shorted drive inductor.

In another aspect, provided is a method for performing a medical procedure using the system.

In an aspect, an articulating probe system comprises: an articulating probe constructed and arranged to articulate in at least one degree of motion and to transition between a flexible state and a rigid state, the articulating probe comprising a plurality of links between a proximal link and a distal link, the plurality of links comprising a plurality of inner links and a plurality of outer links; a feeder assembly in communication with the articulating probe to apply forces on the articulating probe to cause the probe to articulate and to transition between the flexible state and rigid state, and to cause one of the plurality of inner links and plurality of outer links to perform a steering and locking operation and the other of the plurality of inner links and plurality of outer links to perform a locking operation; a plurality of steering cables in communication with the one of the plurality of inner links and plurality of outer links; a locking cable in communication with the other of the plurality of inner links and plurality of outer links; the feeder assembly further comprising: cable bobbins at which proximal ends of the plurality of steering cables and a proximal end of the locking cable are wound; motor assemblies, each corresponding to one of the cable bobbins, for driving the cable bobbins, the motor assemblies including motion resistance mechanisms that substantially prevent rotation of the bobbins as a result of forces transferred through the steering cables and locking cables, as encountered by the articulating probe.

In some embodiments, the motor assemblies comprise: a motor; a gear assembly; and a capstan in communication with the cable bobbin.

In some embodiments, the gear assembly comprises a worm gear assembly.

In some embodiments, the gear assembly comprises at least one of a ratchet and pawl mechanism or a magnetic position holding assembly.

In some embodiments, the motor comprises one of a stepper motor, a closed-loop servomotor, and a DC motor having a shorted drive inductor.

In another aspect, provided is a method for performing a medical procedure using the system.

In an aspect, an articulating probe system comprises: an articulating probe constructed and arranged to articulate in at least one degree of motion and to transition between a flexible state and a rigid state, the articulating probe comprising a plurality of links between a proximal link and a distal link, a feeder assembly in communication with the articulating probe to apply forces on the articulating probe to cause the probe to articulate and to transition between the flexible state and rigid state; a plurality of cables in communication with the plurality of links; the feeder assembly further comprising: cable bobbins at which proximal ends of the plurality of cables are wound; motor assemblies, each corresponding to one of the cable bobbins, for driving the cable bobbins; motor mounts to which the motor assemblies are mounted, the motor mounts being movably coupled to a chassis of the feeder assembly; and load cells in contact with motor mounts for measuring a force applied to the motor mounts.

In some embodiments, the motor assemblies comprise: a motor; a gear assembly; and a capstan in communication with the cable bobbin.

In some embodiments, the load cell measures a force applied to the motor mounts by the cables.

In some embodiments, the feeder assembly further comprises a low-resistance bearing for movably coupling the motor mounts to the chassis of the feeder assembly.

In some embodiments, the feeder assembly further comprises a biasing spring that pre-loads the load cell by applying a biasing force on the motor mounts.

In some embodiments, the feeder assembly further comprises an adjustment screw that ensures contact by the motor mounts against the load cells.

In some embodiments, the feeder assembly further comprises a load cell electronics module for receiving signals generated by the load cell.

In another aspect, provided is a method for performing a medical procedure using the system.

In an aspect, an articulating probe system comprises: an articulating probe constructed and arranged to articulate in at least one degree of motion and to transition between a flexible state and a rigid state, the articulating probe comprising a plurality of links between a proximal link and a distal link, the plurality of links comprising a plurality of inner links and a plurality of outer links; a feeder assembly in communication with the articulating probe to apply forces on the articulating probe to cause the probe to articulate and to transition between the flexible state and rigid state, and to cause one of the plurality of inner links and plurality of outer links to perform a steering and locking operation and the other of the plurality of inner links and plurality of outer links to perform a locking operation; a plurality of steering cables in communication with the one of the plurality of inner links and plurality of outer links; a locking cable in communication with the other of the plurality of inner links and plurality of outer links; the feeder assembly further comprising: cable bobbins at which proximal ends of the plurality of steering cables and a proximal end of the locking cable are wound; motor assemblies, each corresponding to one of the cable bobbins, for driving the cable bobbins; motor mounts to which the motor assemblies are mounted, the motor mounts being movably coupled to a chassis of the feeder assembly; load cells in contact with motor mounts for measuring a force applied to the motor mounts.

In some embodiments, the motor assemblies comprise: a motor; a gear assembly; and a capstan in communication with the cable bobbin.

In some embodiments, the load cell measures a force applied to the motor mounts by the cables.

In some embodiments, the feeder assembly further comprises a low-resistance bearing for movably coupling the motor mounts to the chassis of the feeder assembly.

In some embodiments, the feeder assembly further comprises a biasing spring that pre-loads the load cell by applying a biasing force on the motor mounts.

In some embodiments, the feeder assembly further comprises an adjustment screw that ensures contact by the motor mounts against the load cells.

In some embodiments, the feeder assembly further comprises a load cell electronics module for receiving signals generated by the load cell.

In another aspect, provided is a method for performing a medical procedure using the system.

In an aspect, an articulating probe system comprises: an articulating probe constructed and arranged to articulate in at least one degree of motion and to transition between a flexible state and a rigid state, the articulating probe comprising a plurality of links between a proximal link and a distal link; a feeder assembly in communication with the articulating probe to apply forces on the articulating probe to cause the probe to articulate and to transition between the flexible state and rigid state; and a position sensor at the feeder assembly to determine whether a change in position of the feeder assembly has occurred.

In some embodiments, the position sensor determines whether a change in at least one of a vertical or horizontal position of the feeder assembly has occurred.

In some embodiments, the position sensor determines whether a change in an orientation of the feeder assembly has occurred.

In some embodiments, the position sensor comprises at least one of an accelerometer, a gyroscope or a position switch.

In some embodiments, the articulating probe system further comprises a control system that, in response to a detection of change in position of the feeder assembly by the position sensor, initiates a calibration procedure of the articulating probe system.

In another aspect, provided is a method for performing a medical procedure using the system.

In an aspect, an articulating probe system comprises: an articulating probe constructed and arranged to articulate in at least one degree of motion and to transition between a flexible state and a rigid state, the articulating probe comprising a plurality of links between a proximal link and a distal link, a feeder assembly in communication with the articulating probe to apply forces on the articulating probe to cause the probe to articulate and to transition between the flexible state and rigid state, the feeder assembly comprising: a base assembly including motor assemblies for driving first coupling mechanisms; and a top assembly including second coupling mechanisms in communication with the first coupling mechanisms and for applying the forces on the articulating probe in response to the first coupling mechanisms, the top assembly including the articulating probe, the top assembly removably attachable to the base assembly; and a pivot position between the top assembly and the base assembly about which the top assembly rotates relative to the base assembly during attachment of the top assembly to the base assembly and during removal of the top assembly from the base assembly, the probe at a first position of the feeder assembly and the pivot position at a second position of the feeder assembly, the second position located such that, during removal of the top assembly from the base assembly, the probe of the top assembly moves in an upward direction relative to a patient location.

In some embodiments, during removal of the top assembly from the base assembly, the probe of the top assembly moves in an upward direction relative to a patient location and in a direction away from the patient location.

In some embodiments, during removal of the top assembly from the base assembly, the first coupling mechanisms release from the second coupling mechanisms, thereby releasing forces applied to the articulating probe.

In some embodiments, the base assembly includes a hook and the top assembly includes a heel that communicates with the hook at the pivot position, the hook and the heel forming a locator joint for seating the top assembly at the base assembly.

In another aspect, provided is a method for performing a medical procedure using the system.

In an aspect, an articulating probe system comprises: an articulating probe constructed and arranged to articulate in at least one degree of motion and to transition between a flexible state and a rigid state, the articulating probe comprising a plurality of links between a proximal link and a distal link, a feeder assembly in communication with the articulating probe to apply forces on the articulating probe to cause the probe to articulate and to transition between the flexible state and rigid state, the feeder assembly comprising: a base assembly including motor assemblies for driving first coupling mechanisms; and a top assembly including second coupling mechanisms in communication with the first coupling mechanisms and for applying the forces on the articulating probe in response to the first coupling mechanisms, the top assembly removably attachable to the base assembly at a seated position; and a sensor constructed and arranged to determines whether the top assembly is in the seated position on the base assembly.

In some embodiments, a portion of the sensor is attached to a handle that secures the top assembly to the base assembly.

In some embodiments, the handle includes a cam that secures the top assembly to a latch on the base assembly.

In some embodiments, the articulating probe further comprises a bumper that provides tactile feedback of proper handle engagement.

In some embodiments, the bumper is coupled to the handle.

In some embodiments, the bumper is coupled to the base.

In some embodiments, the bumper is adjustable in height.

In some embodiments, the sensor comprises a magnet and magnetic field sensor assembly.

In some embodiments, the handle is at the top assembly, wherein the magnet is coupled to the handle and wherein the magnetic field sensor assembly is at the base assembly.

In some embodiments, the magnetic field sensor assembly comprises a filter plate that limits the magnetic field emitted by the magnet to a selective region to further increase precision of the magnetic field sensor assembly.

In another aspect, provided is a method for performing a medical procedure using the system.

In an aspect, an articulating probe system comprises: an articulating probe constructed and arranged to articulate in at least one degree of motion and to transition between a flexible state and a rigid state, the articulating probe comprising a plurality of links between a proximal link and a distal link, a feeder assembly in communication with the articulating probe to apply forces on the articulating probe to cause the probe to articulate and to transition between the flexible state and rigid state, the feeder assembly comprising: a base assembly including motor assemblies for driving first coupling mechanisms; and a top assembly including second coupling mechanisms in communication with the first coupling mechanisms and for applying the forces on the articulating probe in response to the first coupling mechanisms, the top assembly including the articulating probe, the top assembly removably attachable to the base assembly, wherein one of the top assembly and base assembly includes a heel and the other of the top assembly and base assembly includes a registration plate at which the top assembly and base assembly interface relative to each other during seating of the top assembly to the base assembly, the heel including a ridge that interfaces with the plate so that the top assembly can rotate slightly about the ridge as it is seated on the base assembly to provide angular play in the seating process.

In some embodiments, the top assembly includes the heel and the base assembly includes the registration plate.

In some embodiments, the articulating probe system further comprises plungers that urge the heel against the plate in a horizontal direction.

In some embodiments, the plungers comprise ball plungers.

In another aspect, provided is a method for performing a medical procedure using the system.

In an aspect, an articulating probe system comprises: an articulating probe constructed and arranged to articulate in at least one degree of motion and to transition between a flexible state and a rigid state, the articulating probe comprising a plurality of links between a proximal link and a distal link, a feeder assembly in communication with the articulating probe to apply forces on the articulating probe to cause the probe to articulate and to transition between the flexible state and rigid state, the feeder assembly comprising: a base assembly including motor assemblies for driving first coupling mechanisms; and a top assembly including second coupling mechanisms in communication with the first coupling mechanisms and for applying the forces on the articulating probe in response to the first coupling mechanisms, the top assembly including the articulating probe, the top assembly removably attachable to the base assembly, the top assembly including: a plurality of cables in communication with the plurality of links; and cable bobbin assemblies at which proximal ends of the plurality of cables are wound, the cable bobbin assemblies corresponding to the second coupling mechanisms and comprising: a bobbin shaft coupled to a bobbin plate; a bobbin including a bore, the bobbin constructed and arranged to rotate about the bobbin shaft; a spring between a bottom of the bobbin and the bobbin plate, the spring biased to urge the bobbin in a direction away from the bobbin plate; and an o-ring about the bobbin shaft, the o-ring constructed and arranged to resist rotation of the bobbin about the bobbin shaft when the bobbin is in a first position whereby the o-ring is seated between the bore and the bobbin shaft.

In some embodiments, the o-ring is constructed and arranged to rest above a top of the bobbin to thereby allow free rotation of the bobbin, when the bobbin is in a second position, in engagement with a corresponding first coupling mechanism of the base.

In some embodiments, the o-ring is constructed and arranged to interface with a top of the bobbin to moderately resist rotation of rotation of the bobbin, when the bobbin is in a third position, under an upward force of the spring and no longer in engagement with a corresponding first coupling mechanism of the base.

In some embodiments, wherein the first position corresponds with a shipment or installation position of the bobbin, wherein the second position corresponds with an operative position of the bobbin and wherein the third position corresponds with a post-operative position of the bobbin.

In some embodiments, the articulating probe system further comprises grooves on an outer surface of the bobbin for locating the proximal end of the cable.

In some embodiments, the articulating probe system further comprises a cable clip over the bobbin that limits cable movement.

In some embodiments, the articulating probe system further comprises a counter bore on the bobbin shaft in which the o-ring is seated.

In some embodiments, the articulating probe system further comprises a counter bore on the bobbin bore.

In some embodiments, the articulating probe system further comprises a washer between the spring and the bottom of the bobbin

In another aspect, provided is a method for performing a medical procedure using the system.

In an aspect, an articulating probe system comprises: an articulating probe constructed and arranged to articulate in at least one degree of motion and to transition between a flexible state and a rigid state, the articulating probe comprising a plurality of links between a proximal link and a distal link, a feeder assembly in communication with the articulating probe to apply forces on the articulating probe to cause the probe to articulate and to transition between the flexible state and rigid state, the feeder assembly comprising: a base assembly including motor assemblies for driving first coupling mechanisms; and a top assembly including second coupling mechanisms in communication with the first coupling mechanisms and for applying the forces on the articulating probe in response to the first coupling mechanisms, the top assembly including the articulating probe, the top assembly removably attachable to the base assembly; a sterile drape constructed and arranged for installation between the base assembly and top assembly, the sterile drape including: a sheet of material; an alignment plate on the sheet of material in alignment with the first and second coupling mechanisms and including pre-formed apertures to operate as pass-throughs for the first and second coupling mechanisms; a removable shield on at least one of the sheet of material in the region of the alignment plate or on the alignment plate or both.

In some embodiments, the removable shield is positioned at a sterile surface of the sheet of material.

In another aspect, provided is a method for performing a medical procedure using the system.

In an aspect, a sterile drape constructed and arranged for installation between a base assembly and top assembly of an articulating probe system, the system including an articulating probe, and a feeder assembly including a non-sterile base having first coupling mechanisms and a sterile top assembly including second coupling mechanisms, the sterile top assembly including the probe, the sterile drape including: a sheet of material; an alignment plate on the sheet of material in alignment with the first and second coupling mechanisms and including pre-formed apertures to operate as pass-throughs for the first and second coupling mechanisms; a removable shield on at least one of the sheet of material in the region of the alignment plate or on the alignment plate or both.

In some embodiments, the removable shield is positioned at a sterile surface of the sheet of material.

In another aspect, provided is a method for performing a medical procedure using the system.

In an aspect, an articulating probe comprises: a plurality of outer links, each outer link comprising a first longitudinal axis, a concave first surface and a convex second surface opposite the first surface; and an inner link channel along the longitudinal axis in a center region thereof; a plurality of inner links, each inner link comprising a first longitudinal axis, a concave first surface and a convex second surface opposite the first surface; and an opening along the longitudinal axis in a center region thereof; the plurality of inner links being positioned in the inner link channels of the plurality of outer links, and slideable relative to the plurality of outer links.

In some embodiments, the plurality of inner links comprises between 10 and 300 inner links, such as between 50 and 150 inner links, such as between 75 and 95 inner links, such as approximately 84 inner links.

In some embodiments, the inner links comprise a length between 0.05" and 1.0", such as between 0.1" and 0.5", such as approximately 0.2".

In some embodiments, the inner links comprise an effective outer diameter of between 0.1" and 1.0", such as an effective outer diameter of between 0.2" and 0.8", such as an effective outer diameter of approximately 0.35".

In some embodiments, the inner links comprise a cable lumen in a central region thereof

In some embodiments, the inner link cable lumen is of a diameter between 0.01" and 0.9", such as a diameter between 0.02" and 0.3", such as a diameter of approximately 0.07".

In some embodiments, the inner link cable lumen comprise an hour-glass profile.

In some embodiments, the concave first surface of the inner links comprises a radius of curvature of between 0.1" to 1.0", such as a radius of between 0.3" and 0.7", such as a radius of approximately 0.55".

In some embodiments, the convex second surface of the inner links comprises a radius of curvature of between 0.1" to 1.0", such as a radius of between 0.3" and 0.7", such as a radius of approximately 0.55".

In some embodiments, a distal-most inner link of the plurality of inner links comprises a tapered convex surface.

In some embodiments, the tapered convex surface of the distal-most inner link of the plurality of inner links lies at an angle relative to the longitudinal axis that is less than an angle of a taper of the convex surface of other inner links of the plurality of inner links..

In some embodiments, the articulating probe comprises more inner links than outer links, such as at least 10% more inner links than outer links, such as at least 50% more inner links than outer links, such as at least 100% more inner links than outer links, such as at least 200% more inner links than outer links, such as at least 300% more inner links than outer links, such as at least 500% more inner links than outer links.

In some embodiments, the plurality of outer links comprises between 5 and 150 outer links, such as between 10 and 100 outer links, such as between 20 and 80 outer links, such as approximately 56 outer links.

In some embodiments, the outer links comprise a length between 0.1" and 2.0", such as between 0.2" and 1.0", such as approximately 0.4".

In some embodiments, the outer links comprise an effective outer diameter of between 0.2" and 2.0", such as an effective outer diameter of between 0.4" and 1.6", such as an effective outer diameter of approximately 0.68".

In some embodiments, the outer links include at least one cable lumen, the cable lumen comprising a diameter between 0.06" and 0.4", such as a diameter between 0.01" and 0.2", such as a lumen with a minimum diameter of approximately 0.047".

In some embodiments, the outer link cable lumens comprise an hour-glass profile.

In some embodiments, a plurality of distal-most outer links comprise material of lubricity that is greater than other outer links of the plurality of links.

In some embodiments, a plurality of distal-most outer links of greater lubricity comprise between 2 and 10 outer links, such as between 2 and 7 outer links.

In some embodiments, one or more outer links comprise an opaque material.

In some embodiments, the distal-most outer link comprises an opaque material.

In some embodiments, the outer links are configured to articulate in a cascading order, in a direction from distal to proximal, during a steering operation.

In some embodiments, the concave first surface of the outer links comprises a radius of curvature of between 0.1" to 1.0", such as a radius of between 0.3" and 0.8", such as approximately 0.57".

In some embodiments, the convex second surface of the outer links comprises a cone with a taper between 5° to 70°, such as a taper of between 10° and 65°, such as a taper of approximately 23°.

In some embodiments, working channels are formed between corresponding recesses at the outer surfaces of the inner links and inner surfaces of the outer links

In some embodiments, working channel recesses of the inner links and/or outer links comprise hour-glass profiles or tapered profiles.

In some embodiments, the hour-glass profile minimize the maximum diameter of the channel or recess, such as would be necessary if the channel or recess had a single, straight taper.

In some embodiments, the outer links are constructed and arranged such that, during a steering operation whereby the outer links undergo articulation, a distal outer link begins to articulate prior to a next-distal-most outer link, in a cascading articulation arrangement.

In some embodiments, a taper angle of the first concave surface of the outer links is varied from link to link in the distal-most outer links to provide the cascading articulation arrangement.

In some embodiments, a variation of the taper angle of the first concave surface of the outer links modifies a mating force between adjacent links to provide the cascading articulation arrangement.

In some embodiments, the taper angle varies from link to link between 10° and 65°, such as increasing from 10° in 1° increments or increasing from 10° in 5° increments

In some embodiments, a characteristic of the outer links is varied from link to link in the distal-most outer links to provide the cascading articulation arrangement, such as a characteristic selected from the group consisting of: other geometric changes such as a geometric change affecting interface force; material change such as a sequential set of lubricity values that decreases; changes in contacting surface area that cause the desired cascade; and combinations of these.

In another aspect, provided is a method for performing a medical procedure using the system.

In another aspect, an articulating probe comprises a plurality of outer links, each outer link comprising: a first longitudinal axis, a concave first surface and a convex second surface opposite the first surface; and an inner link channel along the longitudinal axis in a center region thereof, wherein the outer links are constructed and arranged such that, during a steering operation whereby the outer links undergo articulation, a distal outer link begins to articulate more readily than a next-distal-most outer link, in a cascading articulation arrangement, wherein a taper angle of the first concave surface of the outer links is varied from link to link in the distal-most outer links to provide the cascading articulation arrangement.

In some embodiments, the articulating probe further comprises a plurality of inner links, each inner link comprising a first longitudinal axis, a concave first surface and a convex second surface opposite the first surface; and an opening along the longitudinal axis in a center region thereof, the plurality of inner links being positioned in the inner link channels of the plurality of outer links, and translate relative to the plurality of outer links.

In another aspect, provided is a method for performing a medical procedure using the system.

In an aspect, a method of compensating for extraneous movement in an articulating probe system controlled at a human interface device (HID), comprises: monitoring steering commands as motion presented to the HID by an operator at a sampling rate; integrating the steering commands to produce an integrated command output; and controlling the articulating probe system in response to the integrated steering command.

In some embodiments, the method further comprises applying a scale factor to modify the sampling rate of the monitoring of the steering commands.

In another aspect, provided is a method for performing a medical procedure using the system.

In another aspect, a method of compensating for extraneous movement in an articulating probe system controlled at a human interface device (HID), comprises monitoring a steering motion of an HID manipulated by an operator; generating steering data in response to the monitored steering motion; generating an integrated steering data signal by filtering data corresponding to undesirable motion of the monitored steering motion from the steering data; controlling a movement of an articulating probe in response to the integrated steering data signal, the movement of the articulating probe occurring in response to the steering motion of the HID absent the undesirable motion.

In some embodiments, the steering motion of the human interface device is monitored at a predetermined sampling rate that captures input errors regarding the undesirable motion.

In some embodiments, the method further comprises applying a scale factor to modify the predetermined sampling rate of the monitoring of the steering motion.

In some embodiments, the sampling rate ranges from 1Hz and 10,000Hz for adjusting between fine or small scale factor and a coarse or large scale factor motion control by the human interface device.

In some embodiments, the input signal includes data regarding position, velocity, acceleration, and time of the motion and jitter.

In some embodiments, the data is filtered by removing the jitter from the input signal.

In some embodiments, controlling the movement of the articulating probe comprises outputting a steering command to cable motors at the feeder assembly to activate the cable motors for manipulating the articulating probe system.

In some embodiments, an extraneous movement is caused by jitter or related abrupt, sudden, or other unexpected motion of the human interface device when the human interface device is manipulated by an operator.

In another aspect, provided is a method for performing a medical procedure using the system.

In another aspect, a method for controlling an articulating probe system, comprises receiving, at an input system, an input signal generated in response to a steering motion of a human interface device; converting the steering motion into an input signal; processing the input signal to filter out jitter; outputting the filtered signal to a feeder assembly; and controlling by the feeder assembly a movement of the articulating probe system in response to the filtered signal.

In an aspect, a steering system, comprises an articulating probe system; a feeder assembly that controls the articulating probe system; a human interface device; an input system that receives an input signal generated in response to a steering motion of the human interface device; and a processor that converts the steering motion into an input signal, processes the input signal to filter out jitter, and outputs the filtered signal to the feeder assembly, which controls a movement of the articulating probe system in response to the filtered signal.

In another aspect, provided is a method for performing a medical procedure using the system.

In an aspect, a method of calibrating a control system of an articulating probe system having load cells measuring cable tension in cables controlling steering and locking of first and second link systems of the probe system, comprises: rotate a cable motor assembly to slacken a corresponding cable; measure load cell data under "zero-tension" with cable slackened; and initiate operation of the probe including steering and locking of the probe based on the measured load cell data under "zero-tension"

In some embodiments, the method further comprises determining an orientation of a feeder of the probe system and initiating operation of the probe further in response to the determined orientation.

In some embodiments, the method further comprises performing the calibration operation on a plurality of the cable motor assembly and initiating operation of the probe further in response to multiple measured load cell data under "zero-tension".

In an aspect, a method of calibrating a control system of an articulating probe system having load cells measuring cable tension in cables controlling steering and locking of first and second link systems of the probe system, comprises: monitor a position of a feeder of the probe system; first determine whether a change in position of the feeder system exceeds a first threshold; in event the change in position exceeds the first threshold, second determine whether a change in position of the feeder system is less than a second threshold; in event the change in position is less than the second threshold, perform an adjustment of compensation values of the system; and in event the change in position is greater than the second threshold, initiate a re-calibration of the probe system.

In some embodiments, in the event the change in position is greater than the first threshold and the second threshold, further initiating an alarm signal.

In another aspect, provided is a method for performing a medical procedure using the system.

In an aspect, a method of preventing application of excessive force in an articulating probe system having load cells measuring cable tension in cables controlling steering and locking of first and second link systems of the probe system, comprising: measure cable tension during operation using a load cell; in event cable tension is greater than a first threshold amount, initiate an alarm; determine whether a steering mode is currently performed; and in event steering mode is currently performed, determine whether cable tension is greater than a second threshold amount; in event cable tension is greater than a second threshold amount, determine a direction of steering and whether the direction of steering matches a determined curvature of the probe; in event of match, the steering operation is halted; in event of no match tension is released in the cable; following match determination and compensation, cable tension is measured and compared to a third threshold; and in event cable tension is greater than the third threshold amount, initiate an alarm;

In an aspect, a method of preventing unintended motion in an articulating probe system having load cells measuring cable tension in cables controlling steering and locking of first and second link systems of the probe system, comprises: receive a steering command from an operator; assess the steering command for "aggressive" movement based on at least one of velocity or acceleration of movement; and adjust tension of cables in response to the assessment.

### BRIEF DESCRIPTION OF THE DRAWINGS

The foregoing and other objects, features and advantages of embodiments of the present inventive concepts will be apparent from the more particular description of preferred embodiments, as illustrated in the accompanying drawings in which like reference characters refer to the same elements throughout the different views. The drawings are not necessarily to scale, emphasis instead being placed upon illustrating the principles of the preferred embodiments.
**FIG. 1** is a perspective illustrative view of an articulating probe system, in accordance with the present inventive concepts.
**FIGs. 2A-2C** are graphic demonstrations of a highly articulating probe device, in accordance with the present inventive concepts.
**FIG. 3** is a perspective view of a portion of a tool positioning system, in accordance with the present inventive concepts.
**FIGs. 4A** is a perspective view of a tool support inner tube, in accordance with the present inventive concepts.
**FIG. 4B** is a side view of the interface of the distal end of an introducer, a tool support and an articulating probe, in accordance with the present inventive concepts.
**FIG. 4C** is a perspective view of the interface of the distal end of an introducer, a tool support and an articulating probe, in accordance with the present inventive concepts.
**FIG. 5A** is an exploded design schematic of a detachable feeder top assembly 300 for an articulating probe, in accordance with the present inventive concepts.
**FIG. 5B** is an illustrative internal view of a feeder system, in accordance with the present inventive concepts.
**FIG. 6A** is an illustrative perspective view of a force-transfer driving subassembly of a top assembly, consistent with the present inventive concepts.
**FIG. 6B** is a perspective view of a force-transfer driving subassembly of a top assembly, in accordance with the present inventive concepts.
**FIG. 6C** is an illustrative side-perspective view of a ninety-degree gear transfer subassembly of the force-transfer driving assembly of FIG. 6B, in accordance with the present inventive concepts.
**FIG. 6D** is another illustrative perspective view of a force-transfer driving subassembly of FIG. 6B, in accordance with the present inventive concepts.
**FIG. 6E** is an illustrative perspective view of a bearing mounting block for a lead screw of the force-transfer driving assembly of FIGs. 6A-6B, in accordance with the present inventive concepts.
**FIG. 6F** is an illustrative perspective view of a bearing mounting block for a lead screw of the force-transfer driving assembly of FIGs. 6A-6B, in accordance with the present inventive concepts.
**FIG. 7A** is a perspective view of internal components of a top assembly of a feeder assembly, in accordance with the present inventive concepts.
**FIG. 7B** is a perspective view of the distal end of a feeder assembly with an energy chain removed for illustrative clarity, in accordance with the present inventive concepts.
**FIG. 8** is a schematic illustration of a capstan drive assembly, in accordance with the present inventive concepts.
**FIG. 8A** is a cutaway perspective front view of a feeder assembly, in accordance with the present inventive concepts.
**FIG. 8B** is a close-up cutaway perspective front view of a gear box of a feeder assembly, in accordance with the present inventive concepts.
**FIG. 9** is a partial cutaway perspective front view of a feeder assembly, in accordance with the present inventive concepts.
**FIG. 10** is a schematic view of a safety system, in accordance with the present inventive concepts.
**FIG. 11** is a perspective illustrative view of an articulating probe system, in accordance with the present inventive concepts.
**FIG. 12** is a perspective top view of a base assembly, in accordance with the present inventive concepts.
**FIG. 13** is a bottom view of a top assembly, in accordance with the present inventive concepts.
**FIG. 14** is a perspective cutaway view of a handle of a top assembly of a feeder assembly of an articulating probe system, in accordance with the present inventive concepts.
**FIG. 15** is a perspective cutaway view of a base assembly of a feeder assembly of an articulating probe system, in accordance with the present inventive concepts.
**FIGs. 15A-15C** are perspective views of proximity sensor componentry, in accordance with the present inventive concepts.
**FIG. 16** is a perspective partial cutaway view of a base assembly of a feeder assembly 102 of an articulating probe system, in accordance with the present inventive concepts.
**FIG. 16A** is a section view of a base assembly and of the interaction of a heel and base cutout, in accordance with the present inventive concepts.
**FIG. 16B** is a closeup perspective view of a cam engagement assembly of a base assembly, in accordance with the present inventive concepts.
**FIG. 17A** is a side view of a cable bobbin of a top assembly, positioned in a shipping condition, in accordance with the present inventive concepts.
**FIG. 17B** is a side view of a cable bobbin of a top assembly, positioned in an operating condition, in accordance with the present inventive concepts.
**FIG. 17C** is a side view of a cable bobbin of a top assembly, in a release condition, in accordance with the present inventive concepts.
**FIG. 17D** is a perspective view of a cable bobbin of the top assembly including a cable retention clip according to an embodiment of inventive concepts.
**FIG. 18** is a top view of a sterile drape assembly, in accordance with the present inventive concepts.
**FIG. 18A** is a magnified view of a portion of the drape assembly of FIG. 18, in accordance with the present inventive concepts.
**FIGs. 19A-19F** are various views of embodiments of an inner link, in accordance with the present inventive concepts.
**FIGs. 20A-20F** are various views of an outer link, in accordance with the present inventive concepts.
**FIG. 21** is a side sectional view of a portion of an articulating probe, in accordance with the present inventive concepts.
**FIG. 22** is a side sectional view of the distal portion of an outer link mechanism, in accordance with the present inventive concepts.
**FIGs. 22A and 22B** are magnified views of the conical to spherical interface of two outer links of FIG. 22, in accordance with the present inventive concepts. FIG. 22C is an illustration of system behavior in connection with the embodiment described in connection with FIGs. 22, 22A and 22B.
**FIG. 23** is a view of a steering system, in accordance with the present inventive concepts.
**FIG. 24** is a flow chart of a steering process, in accordance with the present inventive concepts.
**FIG. 25** is a flow chart of a method for performing a calibration, in accordance with the present inventive concepts.
**FIG. 26** is a flow chart of a method for preventing and/or detecting excessive force, in accordance with the present inventive concepts.
**FIG. 27** is a flow chart of a method for detecting and/or reducing unintended motion of an articulating probe, in accordance with the present inventive concepts.
**FIG. 28** is a flow chart of a calibration procedure, in accordance with the present inventive concepts.

### DETAILED DESCRIPTION OF EMBODIMENTS

The terminology used herein is for the purpose of describing particular embodiments and is not intended to be limiting of the inventive concepts. As used herein, the singular forms "a," "an" and "the" are intended to include the plural forms as well, unless the context clearly indicates otherwise. It will be further understood that the terms "comprises," "comprising," "includes" and/or "including," when used herein, specify the presence of stated features, integers, steps, operations, elements, and/or components, but do not preclude the presence or addition of one or more other features, integers, steps, operations, elements, components, and/or groups thereof.

It will be understood that, although the terms first, second, third etc. may be used herein to describe various limitations, elements, components, regions, layers and/or sections, these limitations, elements, components, regions, layers and/or sections should not be limited by these terms. These terms are only used to distinguish one limitation, element, component, region, layer or section from another limitation, element, component, region, layer or section. Thus, a first limitation, element, component, region, layer or section discussed below could be termed a second limitation, element, component, region, layer or section without departing from the teachings of the present application.

It will be further understood that when an element is referred to as being "on" or "connected" or "coupled" to another element, it can be directly on or above, or connected or coupled to, the other element or intervening elements can be present. In contrast, when an element is referred to as being "directly on" or "directly connected" or "directly coupled" to another element, there are no intervening elements present. Other words used to describe the relationship between elements should be interpreted in a like fashion (e.g., "between" versus "directly between," "adjacent" versus "directly adjacent," etc.). When an element is referred to herein as being "over" another element, it can be over or under the other element, and either directly coupled to the other element, or intervening elements may be present, or the elements may be spaced apart by a void or gap.

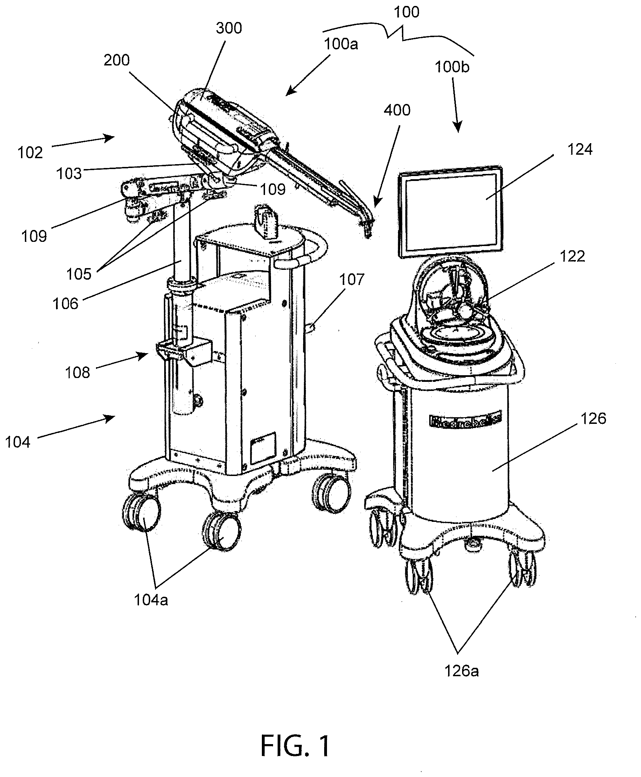

**FIG. 1** is a perspective illustrative view of an articulating probe system 100 according to an embodiment of inventive concepts. In some embodiments, the articulating probe system 100 comprises a feeder unit 100a and an interface unit 100b (also referred to as console 100b). The feeder unit 100a may comprise a feeder assembly 102 mounted to a feeder cart 104 at a feeder arm 106. Feeder arm 106 is adjustable in height, such as via rotation of crank handle 107 which is operably connected to vertical height adjuster 108 which slidingly connects feeder arm 106 to feeder cart 104. Feeder arm 106 can include a plurality of sub-arms that pivot relative to each other at one or more joints 109 that can be locked and/or unlocked via clamps 105. This configuration permits a range of orientations for positioning the feeder assembly 102 relative to a patient location. In some embodiments, one or more feeder supports 103 are attached between feeder arm 106 and feeder assembly 102, such as to partially support the weight of feeder assembly 102 to ease positioning feeder assembly 102 relative to feeder arm 106 (e.g. when one or more lockable joints 109 of feeder arm 106 are in an unlocked position during manipulation). Feeder support 103 can comprise a hydraulic or pneumatic support piston, similar to the gas springs used to support tail gates of automobiles. In some embodiments, two segments of feeder arm 106 are connected with a support piston (not shown but such as a support piston contained within one of the segments), such as to support the weight of feeder assembly 102 or simply base assembly 200.

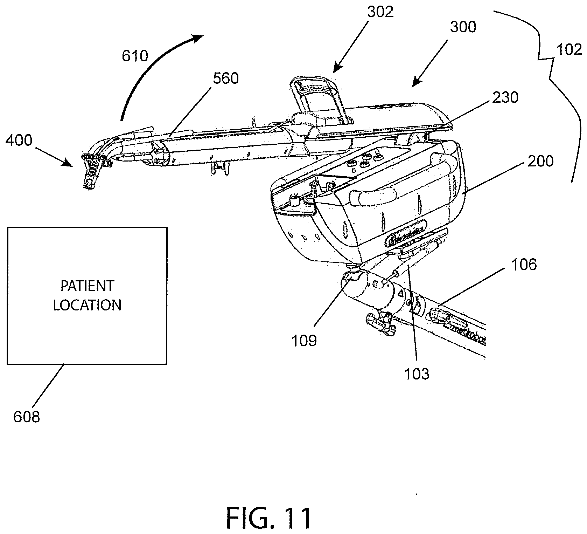

The feeder assembly 102 includes a base assembly 200 and a top assembly 300 that is removably attachable to the base assembly 200. In some embodiments, a first top assembly 300 can be replaced with a second top assembly 300, after one or more uses (e.g. in a disposable manner). In some embodiments, base assembly 200 and top assembly 300 are fixedly attached to each other (see for example, FIG. 11 herein).

The top assembly 300 includes an articulating probe 400 for example comprising a link assembly including an inner link mechanism comprising a plurality of inner links, and an outer link mechanism comprising a plurality of outer links, as described in connection with various embodiments herein. The position, configuration, and/or orientation of the probe 400 are manipulated by a plurality of driving motors, cables, and/or other elements positioned in the base assembly 200. The feeder cart 104 can be mounted on wheels 104a to allow for manual manipulation of its position. Wheels 104a can include one or more locking features used to lock feeder cart 104 in position after a manipulation. In some embodiments, mounting of the feeder assembly 102 to a moveable feeder cart 104 is advantageous, such as to provide a range of positioning options for an operator, versus mounting of feeder assembly 102 to the operating table or other fixed structure.

In some embodiments, the base assembly 200 is operably connected to the interface unit 100b, such connection typically including electrical wires, optical fibers, or wireless communications, for transmission of power and/or data, or mechanical transmission conduits such as mechanical linkages or pneumatic/hydraulic delivery tubes (wired connections not shown). The interface unit 100b includes a human interface device (HID) 122 for receiving tactile commands from a surgeon, technician and/or other operator of system 100, and a display 124 for providing visual and/or auditory feedback. The interface unit 100b can likewise be positioned on an interface cart 126, which is mounted on wheels 126a (e.g. lockable wheels) to allow for manual manipulation of its position.

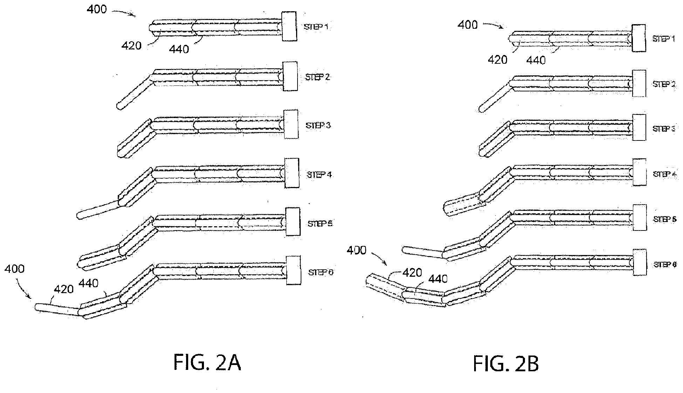

In some embodiments, articulating probe 400 comprises an inner mechanism of articulating links and an outer mechanism of articulating links, such as those described in applicant's co-pending International PCT Application Serial No. PCT/US2012/70924, filed December 20, 2012, the content of which is incorporated herein by reference in its entirety. In some embodiments, articulating probe 400 comprises inner and/or outer links as described herebelow in reference to FIGs. 2A-2C and/or FIGs. 19A-19F and FIGs. 20A-20F.

**FIGs. 2A-2C** are graphic demonstrations of a highly articulating probe device, according to embodiments of the present inventive concepts. A highly articulating robotic probe 400, according to the embodiment shown in FIGs. 2A-2C, comprises essentially two concentric mechanisms, an outer mechanism and an inner mechanism, each of which can be viewed as a steerable mechanism. FIGS. 2A-2C show the concept of how different embodiments of the probe 400 operate. Referring to FIG. 2A, the inner mechanism can be referred to as a first mechanism or inner link mechanism 420. The outer mechanism can be referred to as a second mechanism or outer link mechanism 440. Each mechanism can alternate between being rigid and limp states. In the rigid mode or state, the mechanism is just that - rigid. In the limp mode or state, the mechanism is highly flexible and thus either assumes the shape of its surroundings or can be re-shaped. It should be noted that the term "limp" as used herein does not necessarily denote a structure that passively assumes a particular configuration dependent upon gravity and the shape of its environment; rather, the "limp" structures described in this application are capable of assuming positions and configurations that are desired by the operator of the device, and therefore are articulated and controlled rather than flaccid and passive.

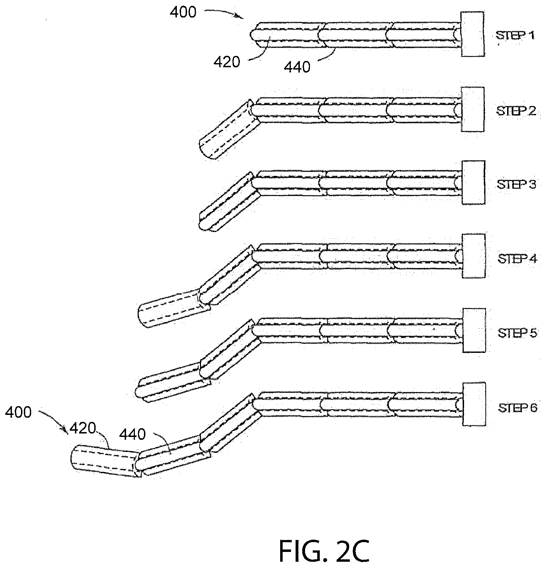

In some embodiments, one mechanism starts limp and the other starts rigid. For the sake of explanation, assume the outer link mechanism 440 is rigid and the inner link mechanism 420 is limp, as seen in step 1 in FIG. 2A. Now, the inner link mechanism 420 is both pushed forward by feeder assembly 102, described herein, and its "head" or distal end is steered, as seen in step 2 in FIG. 2A. Now, the inner link mechanism 420 is made rigid and the outer link mechanism 440 is made limp. The outer link mechanism 440 is then pushed forward until it catches up or is coextensive with the inner link mechanism 420, as seen in step 3 in FIG. 2A. Now, the outer link mechanism 440 is made rigid, the inner link mechanism 420 limp, and the procedure then repeats. One variation of this approach is to have the outer link mechanism 440 be steerable as well. The operation of such a device is illustrated in FIG. 2B. In FIG. 2B it is seen that each mechanism is capable of catching up to the other and then advancing one link beyond. According to one embodiment, the outer link mechanism 440 is steerable and the inner link mechanism 420 is not. The operation of such a device is shown in FIG. 2C, illustrated in a series of steps.

In medical applications, once the probe 400 arrives at a desired location, the operator, typically a surgeon, can slide one or more tools through one or more working channels of outer link mechanism 440, inner link mechanism 420, or one or more working channels formed between outer link mechanism 440 and inner link mechanism 420, such as to perform various diagnostic and/or therapeutic procedures. In some embodiments, the channel is referred to as a working channel that can, for example, extend between first recesses formed in a system of outer links and second recesses formed in a system of inner links. Working channels may be included on the periphery of probe 400, such as working channels comprising one or more radial projections extending from outer link mechanism 440, these projections including one or more holes sized to slidingly receive one or more tools.

In addition to clinical procedures such as surgery, probe 400 can be used in numerous applications including but not limited to: engine inspection, repair or retrofitting; tank inspection and repair; surveillance applications; bomb disarming; inspection or repair in tightly confined spaces such as submarine compartments or nuclear weapons; structural inspections such as building inspections; hazardous waste remediation; biological sample and toxin recovery; and combination of these. Clearly, the device of the present disclosure has a wide variety of applications and should not be taken as being limited to any particular application.

Inner link mechanism 420 and/or outer link mechanism 440 are steerable and inner link mechanism 420 and outer link mechanism 440 can each be made both rigid and limp, allowing probe 400 to drive anywhere in three-dimensions while being self-supporting. Probe 400 can "remember" each of its previous configurations and for this reason, probe 400 can retract from and/or retrace to anywhere in a three dimensional volume such as the intracavity spaces in the body of a patient such as a human patient.

The inner link mechanism 420 and outer link mechanism 440 each include a series of links, i.e. inner links and outer links respectively, that articulate relative to each other. In some embodiments, the outer links are used to steer and lock the probe, while the inner links are used to lock the probe. In "follow the leader" fashion, while the inner links are locked, the outer links are advanced beyond a distal-most inner link. The outer links are steered into position by the system steering cables, and then locked by locking the steering cables. The cable of the inner links is then released and the inner links are advanced to follow the outer links. The procedure progresses in this manner until a desired position and orientation are achieved. The combined inner and outer links include working channels for temporary or permanent insertion of tools at the surgery site. In some embodiments, the tools can advance with the links during positioning of the probe. In some embodiments, the tools can be inserted through the links following positioning of the probe.

One or more outer links can be advanced beyond the distal-most inner link prior to the initiation of a operator controlled steering maneuver, such that the quantity extending beyond the distal-most inner link will collectively articulate based on steering commands. Multiple link steering can be used to reduce procedure time, such as when the specificity of single link steering is not required. In some embodiments, between 2 and 20 outer links can be selected for simultaneous steering, such as between 2 and 10 outer links or between 2 and 7 outer links. The number of links used to steer corresponds to achievable steering paths, with smaller numbers enabling more specificity of curvature of probe 400. In some embodiments, an operator can select the number of links used for steering (e.g. to select between 1 and 10 links to be advanced prior to each steering maneuver).

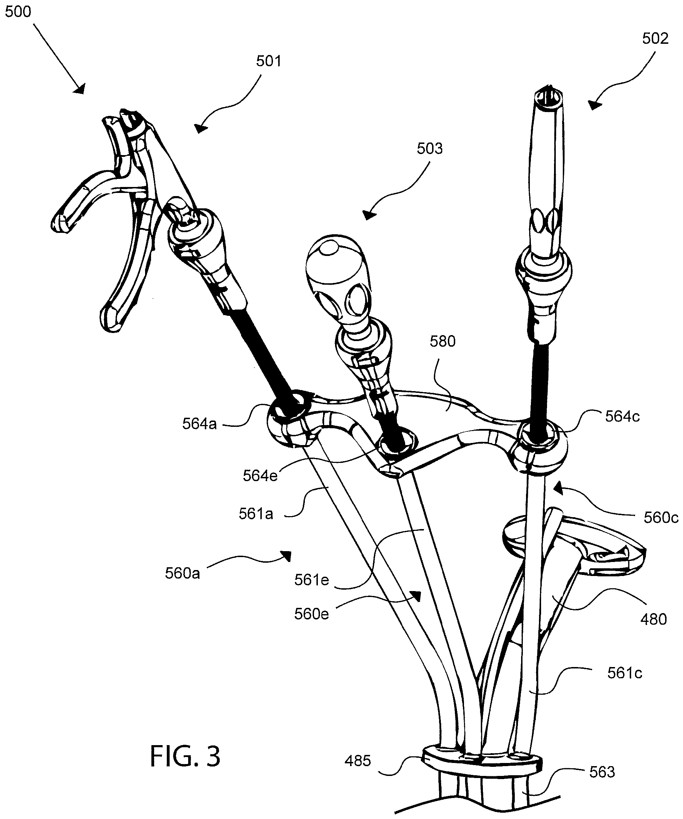

**FIG. 3** is a perspective view of a portion of a tool positioning system 500 in accordance with the inventive concepts. The tool positioning system 500 comprises at least an introduction device, or introducer 480, and one or more tools supports 560, such as a first tool support 560a and a second tool support 560c. In some embodiments, system 500 includes at least three tool supports 560, such as when system 500 further comprises a third tool support 560e. Tool supports 560 are each constructed and arranged to slidingly receive a tool, for example, a shaft of a tool.

The introducer 480 can be constructed and arranged to slidingly receive an articulating probe such as the articulating probe 400, and support, stabilize, and/or guide the articulating probe to a region of interest. The region of interest may be a lumen of a body of a patient (P), such as a cavity at the patient's head (H), e.g., a nose or mouth, or an opening formed by an incision. In clinical applications, typical regions of interest can include but not be limited to the esophagus or other locations within the gastrointestinal tract, the pericardial space, the peritoneal space, and combinations thereof. The region of interest may alternatively be a non-human region, such as a mechanical device, a building, or another open or closed environment in which the system 500 can be used.

In the embodiment of FIG. 3, three tools 501, 502, 503 are inserted into tool supports 560a, 560c and 560e, respectively. A single operator can operate tool positioning system 500, including any or all three tools 501, 502, 503. Alternatively, two or more operators can operate tool positioning system 500, including any or all three tools 501, 502, 503.

Three tool supports 560a, 560c, 560e extend between a base 485 and a connector 580. Connector 580 can connect and/or otherwise provide a stabilizing force between two or more tool supports 560 as shown. Each of tool supports 560a, 560c and 560e can include a funnel-shaped opening, 564a, 564c and 564e respectively, on their proximal end, such as to create a smooth entry for tool insertion. The base 485 includes a collar having first, second, and third openings aligned with the first, second, and third tool supports 560a, 560c, 560e, respectively. The guide elements 561a, 561c, 561e (generally, 561) of the first, second, third and tool supports 560a, 560c, 560e, respectively, can extend through the first, second, and third openings so that mid-portions of the guide elements 561 are positioned in the openings during operation. The base 485 can include a fourth opening for receiving introducer 480. In some embodiments, introducer 480 comprises base 485.

At least one tool 501, 502, 503 can have a shaft, shown inserted into tool supports 560a, 560c and 560e, respectively, constructed and arranged to be slidingly received by one or more tool supports 560. One or more of tools 501, 502, 503 can be selected from the group consisting of: suction device; ventilator; light; camera; grasper; laser; cautery; clip applier; scissors; needle; needle driver; scalpel; RF energy delivery device; cryogenic energy delivery device; and combinations thereof. A tool 501, 502, 503 can include a rigid and/or a flexible tool shaft.

The connector 580 is attached to first, second, and third tool supports 560a, 560c, 560e and can be constructed and arranged to maintain a relative distance between the tool supports 560a, 560c and/or 560e. The connector 580 can be fixedly attached to one or more of the tool supports 560. Alternatively, the connector 580 can be rotatably attached to one or more of the tool supports 560. The connector 580 can be constructed and arranged to be attachable to and/or detachable from the tool supports 560, such as when multiple connectors 580 (e.g. with different separation distances and/or other differences) are provided in system 500 such that different arrangements of tool supports 560 can be accomplished.

The base 485 can be fixedly attached to one or more of the tool supports 560. Alternatively, the base 485 can be rotatably attached to one or more of the tool supports 560. A gimbal (not shown) can be positioned at the base 485 and rotatably engage one or more guide elements 561 at the base 485.

A single operator can operate one or more of: the tool 501 extending from the first tool support 560a, the tool 502 extending from the second tool support 560c, and/or the tool 503 extending from the third tool support 560e, for example, from a single operator location. Alternatively, one operator can operate two tools of the tools 501, 502, 503, and another operator can operate the remaining tool of the tools 501, 502, 503.

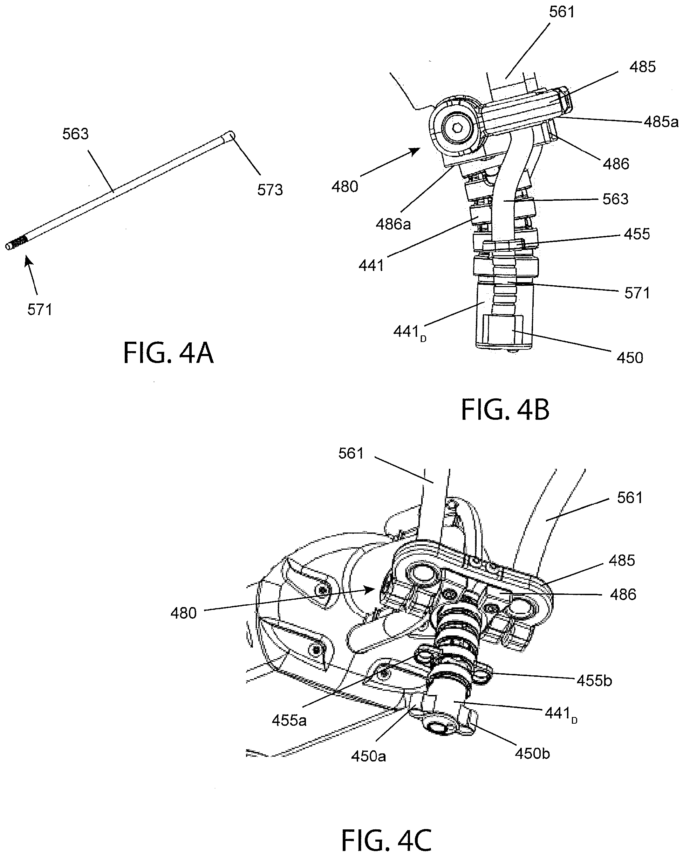

**FIG. 4A** is a perspective view of a tool support inner tube, in accordance with embodiments of the present inventive concepts. FIG. 4B is a side view of the interface of the distal end of an introducer, a tool support and an articulating probe, in accordance with embodiments of the present inventive concepts. FIG. 4C is a perspective view of the interface of the distal end of an introducer, a tool support and an articulating probe in accordance with embodiments of the present inventive concepts.

Referring to FIGS 4A, 4B and 4C, and with reference to the tool positioning system 500 of FIG. 3, a distal end of an introducer 480 and its base 485 are shown. A distal outer link 441_{D} of articulating probe 400 includes first and second distal side ports 450a, 450b, at which tools can be slidingly supported. Tool support guide element 561 extends from a top portion of the base 485. A tool support inner tube 563 (see FIGs. 3 and 4A) is slidably positioned within the tool support guide element 561 (note that tool support inner tubes 563 have been removed from FIG. 4C for illustrative clarity). Inner tubes 563 and/or guide element 561 may have a circular cross-section, or elliptical cross-section, or other shape permitting the tubes to operate according to embodiments described herein. In some embodiments, the tool support inner tube 563 is anchored (e.g. fixedly, rotatably or otherwise attached), at its distal end, to the respective one of the first and second distal side ports 450a, 450b. In this manner, as the distal outer link 441_{D} of the probe is advanced (e.g. in a longitudinal direction), the tool support guide element 561 remains fixed in position, while the tool support inner tube increases in length of extension from the base 485.

In some embodiments, one or more intermediate outer links 441 can include one or more side ports, such as the two intermediate side ports 455a, 455b shown (generally, intermediate side ports 455), through which the tool support inner tube 563 can slidingly pass. The intermediate side ports 455 operate as a locator and/or structural support for the tool support inner tube to prevent inadvertent buckling or bending of the tool support inner tube 563, and/or to otherwise provide a smooth translation of one or more elongate tool shafts or other filaments passing through the tool support 560.

In some embodiments, the tool support inner tube 563 can include a flexibility enhancement feature at its distal portion 571. In the present embodiment, the tool support inner tube 563 includes rib features on distal portion 571, the indents of the ribs being of reduced outer diameter. The tube 563 can formed of plastic, such as polytetrafluoroethylene (PTFE), polyether block amide (Pebax®) or the like. Alternatively, the tube 563 can be formed of two tubes with a coil or braid in between (e.g. a metal or plastic coil or braid). Here, the two tubes can be formed of PTFE tube and a Pebax® material, or the like. The tube 563 can include a liner, formed of PTFE or the like. Such ribbing provides for enhanced flexibility in the distal region of the tool support inner tube 563. In some embodiments, the ribbed portion has a different material composition than the main body portion (e.g. a more flexible material or other more flexible material composition), a portion that has walls that are relatively thinner than the main body portion and/or other applicable mechanisms for enhancing flexibility.

Full steering capability of the distal outer link 441D and proximate outer links 441 of the articulating probe 400 is highly desired for proper probe operation. By enhancing the relative flexibility of the tool support inner tube 563, any interference with steering capability by the tube 563 is mitigated or prevented.

A proximal end of the tool support inner tube 563 can include a funnel-shaped feature 573 to aid in tool insertion.

In some embodiments, the base 485 of the introducer 480 includes a flange 486 or the like that projects from the undersurface 485a of the base 485. The flange 486 is positioned to communicate with (e.g. extend) the channel of the introducer 480, through which the articulating probe 400 passes. In this manner, the flange 486 provides additional support for probe 400 proximate the point at which it leaves or otherwise extends from introducer 480. With reference to FIG 4B, it can be seen that the surface 486a of flange 486 at which probe 400 exits is more distal (e.g. lower on the page) than the surface of base 485 at which tool support inner tube 563 exits. In this manner, probe 400 is further supported, reducing its moment arm relative to the point at which it exits the introducer 480. At the same time, the exit location of tool support inner tube 563 is maintained by not passing through flange 486 and is instead adjacent or external to flange 486, such as to allow for angulation of a tool passing through inner tube 563 at a pivot location proximal to the exit location of probe 400 from flange 486. Flange 486 can comprise an attachable component (e.g. attachable to the remainder of introducer 480), or it can be fixedly attached (e.g. a single piece construction of introducer 480). In some embodiments, multiple attachable flanges 486 are provided to provide different configurations for the support of probe 400.

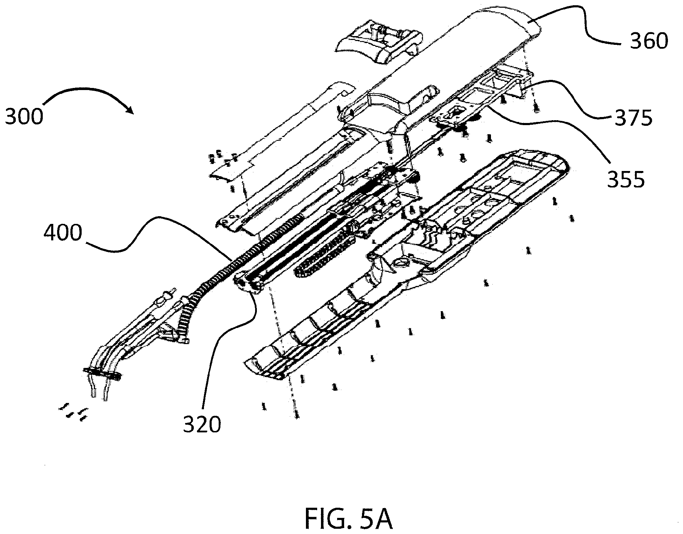

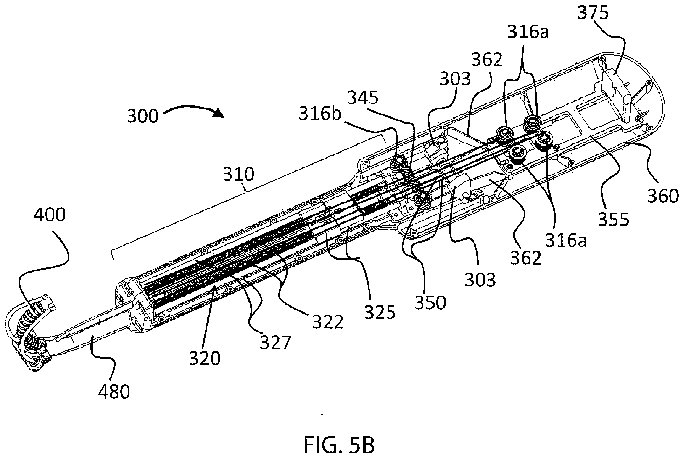

**FIG. 5A** is an exploded design schematic of a detachable feeder top assembly 300 for an articulating probe, such as articulating probe 400 described herein, according to an embodiment of inventive concepts. **FIG. 5B** is an illustrative internal view of an assembled feeder system according to an embodiment of inventive concepts. In an embodiment, the top assembly 300 includes a housing 360 having a stabilization plate 355, at which cable bobbins 316a are positioned. Housing 360 is typically an injection molded, plastic housing, such as a reinforced plastic housing. In an embodiment, the stabilization plate 355 is mounted to housing 360 proximate one or more reinforced housing ribs 362. In an embodiment, cables 350 extend through an articulating probe 400 comprising both inner and outer links (e.g., the links of inner link mechanism 420 and outer link mechanism 440 of FIGs. 2A - 2C). In an embodiment, the cables 350 can be used to steer and/or reversibly tighten to "lock"/stiffen either or both of the inner link mechanism 420 or outer link mechanism 440 such as is described herein. In an embodiment, one or more cables 350 can be used to lock the links and two or more cables 350 can be used to steer the links. For example, three cables 350 can be designated for steering the links of outer link mechanism 440 of FIGs. 2A-2C in three dimensions. These three cables 350 can also be used for locking the outer link mechanism 440. The remaining cable(s) 350 can be used for locking the links of inner link mechanism 420. In an embodiment, when using cables 350 for locking, the forces applied can be distributed equally or unequally among cables 350. For example, if a 36 lb force is applied for locking the outer link mechanism 440 connected to three cables 350, a force of 12 lbs can be applied equally to each of the connected cables. In an embodiment, three of the bobbins 316a are configured to control the outer links, such as to steer, feed cable for articulating probe 400 advancement, retract cable for probe 400 retraction, transition probe 400 from a limp to a rigid state (e.g. to lock), and to transition probe 400 from a rigid to a limp state (e.g. to become flexible). In this embodiment, one bobbin 316a is typically used to control the inner links, such as to feed cable for probe 400 advancement, retract cable for probe 400 retraction, transition probe 400 from a limp to a rigid state (e.g. to lock), and to transition probe 400 from a rigid to a limp state (e.g. to become flexible). In some embodiments, the forces exerted by the bobbins 316a on cables 350 can exceed 1, 10, 30 and/or 50 pounds, such as to lock the attached inner or outer links of probe 400. In configurations in which four cables are used to steer and lock probe 400, collective forces exerted by the bobbins 316a can exceed 95 pounds, such as when 50 pounds is applied to lock the inner links (e.g. with a single cable) and 15 pounds per cable is used to lock the outer links (e.g. with three cables). In various embodiments, the amount of force applied is related to the size (including diameter and length) of the links of the inner link mechanism 420 and outer link mechanism 440 and also to the smoothness of the steering of the links. Greater force may be necessary to lock and stabilize a set of larger and/or longer links, including when the links are extended or retracted with respect to each other.

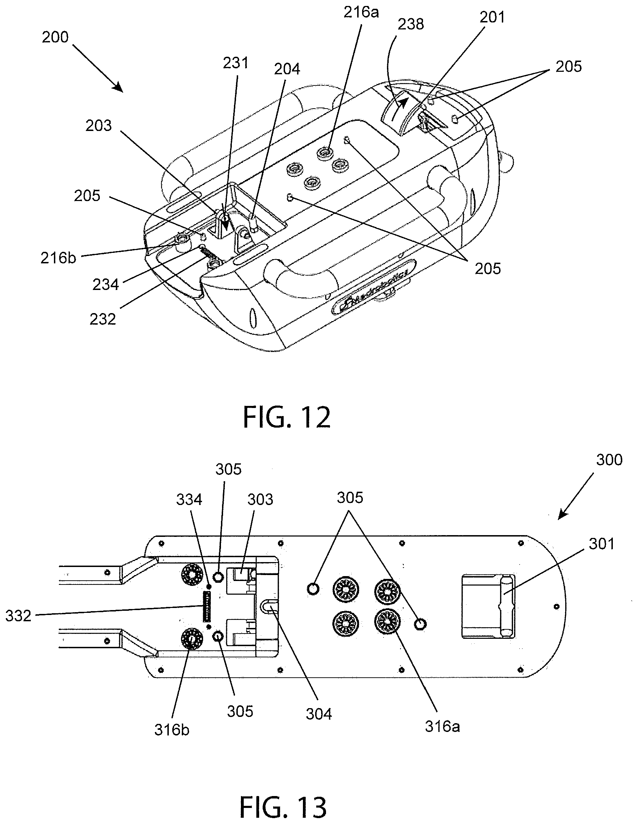

A heel plate 375 (also referred to as heel 301 herein) is fixedly attached to the stabilization plate 355 and can lockably engage with base assembly 200 as described herein. Cams 303 are also attached to the housing 360 which are arranged to lockably engage with base assembly 200. In an embodiment, cams 303 can articulate and are spring loaded, so as to rotate downward upon engaging latch prongs (such as engagement assembly 203 of FIG. 12). In an embodiment, the spring loaded cams 303 provide up to about 20 pounds of tension, but is not limited thereto. The heel plate 375 and cams 303 interlock with base assembly 200 and thereby stabilize and aid in the resistance of undesired motion, including lateral motion, of the feeder system and base assembly 200 during the transfer of power (e.g. cable-applied force) to the probe 400 such as via bobbins 316a. As described herein, the top assembly 300 can be configured to be detachable from base assembly 200, such as to be cleaned or replaced with another top assembly 300 (e.g. a new, sterile top assembly 300), such as when probe 400 is exposed to biological or toxic materials.

A carriage drive segment 310 is attached distally to a reinforced introducer 480, through which probe 400 extends. Introducer 480 can be used for guiding the probe 400's initial path through or toward a target area such as, for example, when introducer 480 comprises an outer surface similar to a body cavity shape found in a majority of patients. Probe 400 can be configured to rapidly advance through introducer 480, prior to fine motion control used after probe 400 exits introducer 480, for example, when performing a medical procedure on a patient using the probe 400.

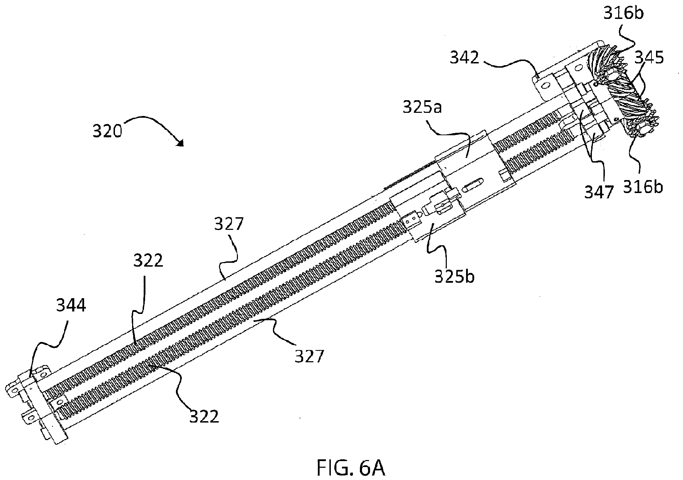

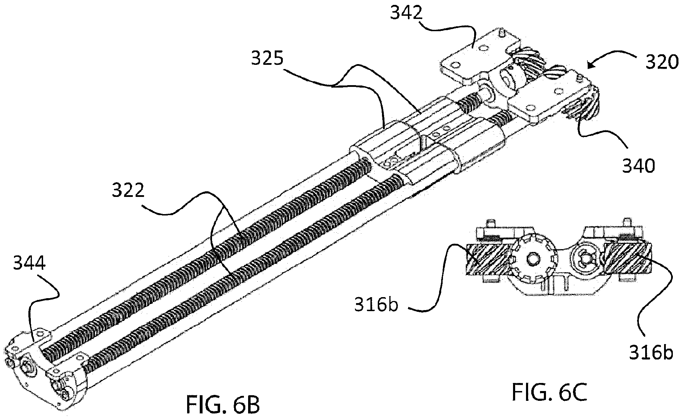

**Referring to** **FIGs. 5A****,** **5B** **and** **6A****,** an illustrative perspective view of a force-transfer driving subassembly 320 of the top assembly 300 is shown. Top assembly 300 includes a carriage drive segment 310 which is configured to independently drive two carriage assemblies, carriages 325, along two lead screws 322. Lead screws 322 can comprise a pitch configured to cause lead screws 322 to be non-backdrivable. In an embodiment, one carriage 325b drives an outer link mechanism 440 and one carriage 325a (independent of carriage 325b) drives an inner link mechanism 420 as described, for example, with respect to FIGs. 2A-2C. The lead screws 322 are driven by a ninety-degree gear assembly which may include gears 316b and gears 345, and/or other related elements for rotating the lead screws 322. In an embodiment, gears 316b and 345 include helical threads so as to increase overall contact between them and further stabilize force transfer between base assembly 200 and probe 400. Lead screws 322 are secured within bearing mounting blocks 342 and 344 that are mounted to housing 360. In an embodiment, bearing mounting block 342 includes thrust bearings 347 for further stabilizing the force transfer between gears 345 and lead screws 322. In an embodiment, carriages 325a, b (generally, 325) include grooves to slidably ride upon guide rails 327, which aid in ensuring linear movement of carriages 325 relative to the rotating motion of the guide rails 327 and providing additional stabilization of the subassembly 320, top assembly 300, and probe 400, so as to resist undesired movement during force-transfer, such as undesired torqueing or compression of top assembly 300. Guide rails 327 can further prevent undesired relative movement between the carriages 325, particularly when unequal forces are applied to them. In an embodiment, guide rails 327 are slidingly received and fixed within bearing mounting blocks 344 and 342 in order to maintain substantially parallel configuration to maintain stability of the top assembly 300. In an embodiment, guide rails 327 are configured to have square, rectangular, round, slotted, or other various cross sectional shapes configured to slidingly engage a receiving portion of carriages 325. In one embodiment, guide rails 327 have a rectangular cross section configured to prevent undesired twisting along one or more axes of top assembly 300 (e.g. the major axis of top assembly 300). The dual screw and rail configuration helps, in particular, to resist twisting and bending of the feeder system. In an embodiment, subassembly 320 is a separate subassembly that is secured into the housing 360 to minimize the deflection of the housing during force transfer, such as when housing 360 comprises a plastic, injection-molded housing. In an embodiment, the carriages 325 include reinforced bushings to engage with the lead screws and/or rails. In an embodiment, the bushings are coated and/or filled with Teflon or a similarly lubricious material. **FIG. 6B** is a perspective view of a force-transfer driving subassembly 320 of the top assembly 300 according to an embodiment of inventive concepts. **FIG. 6C** is an illustrative side-perspective view of a ninety-degree gear transfer subassembly of the force-transfer driving subassembly 320 of FIG. 6B.

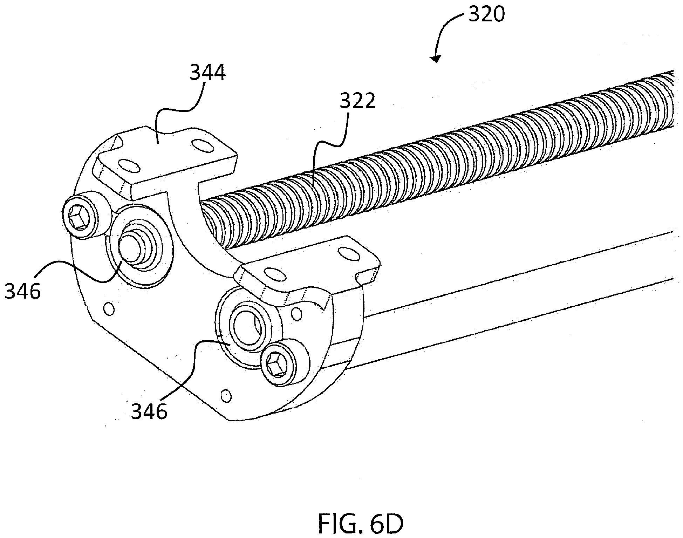

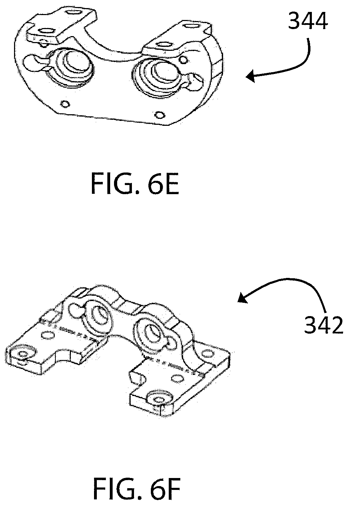

**FIG. 6D** is another illustrative perspective view of a force-transfer driving subassembly 320 of FIG. 6B, with one lead screw 322 and other components removed for illustrative clarity. In an embodiment, the mounting block 344 includes spherical bearings 346 to help ensure proper alignment between the lead screw 322 and the bearing mounting block 344. **FIG. 6E** is an illustrative perspective view of a bearing mounting block 344 for a lead screw (not shown) of the force-transfer driving assembly of FIGs. 6A-6B according to an embodiment of inventive concepts.

**FIG. 6F** is an illustrative perspective view of a bearing mounting block 342 for a lead screw 322 of the force-transfer driving subassembly 320 of FIGs. 6A-6B. As discussed above, in an embodiment, bearing mounting block 342 includes thrust bearings 347 for further stabilizing the force transfer between gears 345 and lead screws 322.

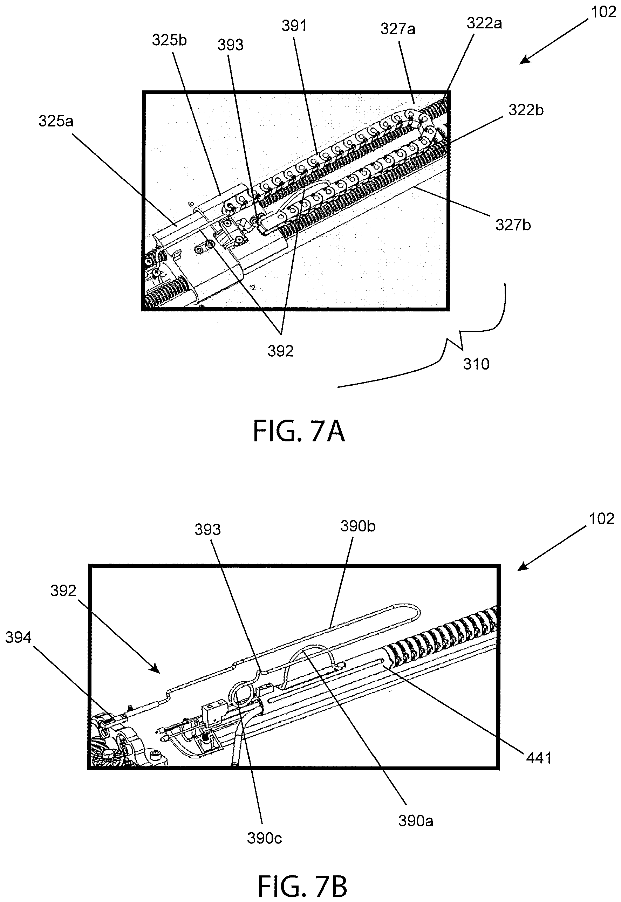

**FIG. 7A** is a perspective view of internal components of a top assembly 300 of a feeder assembly 102 in accordance with inventive concepts. Feeder assembly 102 includes a carriage drive segment 310 including first and second carriages 325a, 325b which glide along first and second guide rails 327a, 327b. First carriage 325a communicates with a first lead screw 322a, and a second carriage 325b communicates with a second lead screw 322b. In this manner, rotation of the lead screw 322a, 322b is translated to linear movement of the corresponding carriage 325a, 325b for driving the carriage 325a, 325b in a linear path along the guide rails 327a, 327b. In some embodiments, the first carriage 325b comprises an inner carriage in communication with inner link mechanism 420 of probe 400. The second carriage 325b comprises an outer carriage in communication with outer link mechanism 440 of probe 400. The carriages 325a, 325b (generally, 325) are each coupled to a proximal-most link of the inner and outer link mechanisms 420, 440 so that the mechanisms can be independently advanced and retracted in a longitudinal direction. An energy chain 391 is coupled at a first end to a fixed (non-moving) portion of top assembly 300, and at a second end to the second carriage 325b. Segments of the energy chain 391 extend and retract as carriage 325b moves relative to non-moving portions of top assembly 300. The energy chain 391 can be employed as a protective mechanism for wires and flexible filaments that extend through the links of probe 400 from the feeder assembly 102. The energy chain 391 can comprise a chain-like construction having a central aperture for receiving flexile filaments such as conduit 392. In some embodiments, energy chain 391 provides a bias such that it changes curvature while remaining substantially in a single plane.

In some embodiments, the conduit 392 comprises a camera cable over which electrical and optical signals, for example, data signals, power signals, and the like, are transferred between a camera optic mounted to a distal link of the inner and outer link mechanisms and the base assembly 200. As the probe extends in a distal direction during a procedure, additional cable is allowed to freely pass in the distal direction, so as not to interfere with steering of the probe. As the probe is steered in a particular orientation that is off-axis, relative to the axis of extension, additional cable is required to be fed into the probe. In addition, in some embodiments, the number of outer links used for a steering maneuver can vary, as described herein. In such a case, the cable is freely allowed to pass through the links to the feeder, and the length of the cable passing through the probe varies in response to the number of links used in the steering maneuver. Accordingly, the conduit 341 can include one or more service loops 390a, 390b, 390c (see FIG. 7B). The service loops 390a, 390b, 390c provide for additional slack conduit 392 that can be fed into and removed from the probe, depending on the position of the distal end of the probe relative to the feeder base.

**FIG. 7B** is a perspective view of the distal end of the feeder assembly 102 with the energy chain removed for illustrative clarity. In some embodiments, a first service loop 390a in the cable provides for maximum steering of the current quantity of distal-most outer links used in a steering maneuver (e.g. as selected by an operator). The first service loop 390a includes a bend that permits for free movement of the cable into and out of the probe during the steering maneuvers. In some embodiments, conduit 392 comprises a camera cable and the first service loop 390a is coupled at a first end at a camera optic positioned in the distal-most outer link 441D of probe 400 and is coupled at a second end 393 to the second carriage 325b. The length of the first service loop 390a is chosen to support all possible configurations of articulating probe 400 that could possibly be encountered during a cumulative set of steering maneuvers (e.g. to support steering of the scope in its minimum bend radius at furthest advancement of outer link mechanism 440). In this manner, steering operations can occur in probe 400 without interference from tension in the conduit 392 due to insufficient conduit length. In the present example embodiment, the first service loop 390a passes through an aperture in a most-proximal outer link 441 of probe 400. In some embodiments, the first service loop 390a comprises third service loop 390c as shown (e.g. comprising multiple physical loops of conduit 392 collectively configured to support all potential steering maneuvers of probe 400).

In some embodiments, a second service loop 390b in conduit 392 provides for advancement and retraction of probe 400. The second service loop 390b includes a loop portion that permits for free movement of second carriage 325b (e.g. while driving the outer link mechanism 440). In some embodiments, conduit 392 comprises a camera cable and the second service loop 390b is coupled at a first end at a camera connector 394 to a camera circuit board and is coupled at a second end 393 to the second carriage 325b. The length of the second service loop 390b is chosen to be longer than the maximum distance of linear translation of the second carriage 325b, such as to accommodate all ranges of translation of second carriage 325b. As shown in FIG. 7B, the second service loop 390b can be protected and seated by the energy chain 391.

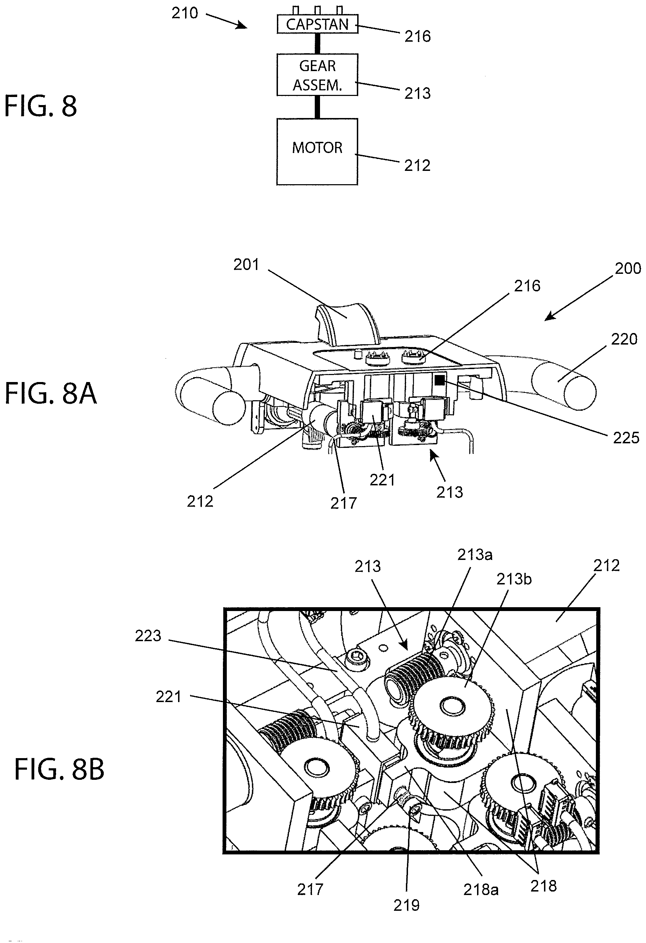

**FIG. 8** is a schematic illustration of a capstan drive assembly, in accordance with the present inventive concepts. FIG. 8A is a cutaway perspective front view of a feeder assembly, in accordance with the present inventive concepts. FIG. 8B is a close-up cutaway perspective front view of a gear box of a feeder assembly, in accordance with the present inventive concepts.

**Referring to** **FIG. 8****,** in some embodiments, a plurality of drive assemblies 210 are provided in the base assembly 200 of the feeder assembly 102. Each drive assembly 210 includes, in some embodiments, a motor 212, a gear assembly 213 and a capstan 216. The capstan 216 is constructed and arranged to mate with a corresponding bobbin on the top assembly 300. In alternative embodiments, the drive assembly 210 can include a bobbin, rather than a capstan, in which case, top assembly 300 includes a corresponding capstan.

The drive assemblies 210 and corresponding capstans 216 drive bobbins 316a on top assembly 300, the bobbins in turn driving cables and on top assembly 300, the cables used to control the operation of probe 400. In various embodiments, motor 212 can comprise any of a number of suitable motor types, including, but not limited to, a brushless DC motor, a stepper motor, a closed-loop servo motor. In various embodiments, a motor linkage encoder or position sensor may be included (e.g. in motor 212 and/or gear assembly 213) for providing closed-loop operation. The gear assembly 213 may comprise a mechanical assembly, for example, providing up to a 20:1 gear ratio, which can be connected to motor 212 to correspondingly reduce the rotational displacement provided by motor 212 (e.g. and corresponding increase the torque provided). Additionally or alternatively, motor 212 itself may optionally include the gear assembly, for example providing a gear reduction of up to 16:1.

In accordance with the present inventive concepts, motor 212 and gear assembly 213 can be configured to resist cable motion at the bobbins. In this manner, the bobbins rotate only when driven by the motor 212, and resist other inherent motion that may otherwise be transferred through the cable from probe 400. In this manner, the motors 212 are substantially resistant to back-driving by forces applied by the steering cables. With enhanced motion resistance capability, the motors 212 can be powered down when not in use, for example, between motion cycles (e.g. steering and/or translation maneuvers), conserving energy, reducing heat output and extending lifespan of drive assembly 210. Also, when an external force is applied to probe 400, for example, when probe 400 is in contact with tissue, there is no need to power the motors of the probe to resist undesired probe motion.

Such enhanced motion resistance can be achieved in any of a number of approaches. In some embodiments, a worm gear gearing mechanism can be employed for drive assembly 210. Such worm-gear gearing mechanisms are inherently non-backdrivable. In other embodiments, a stepper motor having a suitable retention force can be applied. In another embodiment, a DC motor with a short-circuited drive inductor can be employed, since any rotation relative to the motor magnets is resisted in this configuration. In other embodiments, mechanical gears with anti-rotation elements, for example pawls or ratchets, can be employed. In other embodiments, magnetic-based position-holding assemblies can be employed to provide a motor retention force.

**Referring to** **FIG. 8A and 8B****,** base assembly 200 includes a base handle 220 for positioning the base, a motor 212, a gear assembly 213 and a capstan 216. Gear assembly 213 comprises a worm 213a and a mating gear 213b. In the close-up view of FIG. 8B, it can be seen that motor 212 drives worm gear assembly 213. The threads of the worm 213a mesh with gear 213b for driving the capstan (not shown) and corresponding bobbin. Any counter-rotational force of the gear 213b applied by the cable attached to the corresponding bobbin is resisted by the interface of gear 213b and worm 213a. In this manner, the cable is locked in place due to the inherent locking (i.e. anti-backdrivable nature) of the mechanical relationship between the worm 213a and gear 213b.

In some embodiments, the motor 212 is attached to the chassis of the base assembly 200 at motor mount 218. In some embodiments, the motor mounts 218 are each rotatably mounted to the chassis of the base assembly 200 and rotate about the axle of gear 213b. In some embodiments, the motor mount 218 is constructed and arranged to rotate with minimal resistance. In some embodiments, the motor mount 218 rotates on a low resistance bearing. In some embodiments a portion 218a of the motor mount 218 rotates to interface with a load cell 221 mounted to the chassis of the base assembly 200. The load cell 221 includes a cable 223 for providing load information to feeder unit 100a and/or interface unit 100b.

In this manner, motor mount 218 engages with load cell 221 to provide a measured force that can be correlated to cable tension in the cable applied to the bobbin 316a corresponding with the given motor 212. The cable tension applies a torsional force on the bobbin and the associated engaged capstan. This in turn applies a torque to the driving gear 213b (e.g. of gear assembly 213) and thus motor 212 and motor mount 218. The motor mount 218 tends to rotate as cable tension is applied. Such rotation applies force to the load cell 221. In this manner, the force measured at the load cell can be correlated to cable tension.

In some embodiments, the interface of the motor mount 218 and load cell 221 can include an adjustment screw 219 for ensuring and/or adjusting contact therebetween. A biasing spring can be further included for ensuring a minimum load is always present on the load cell. This configuration avoids load cell measurements near zero force, which can be a desired avoidance in such applications.

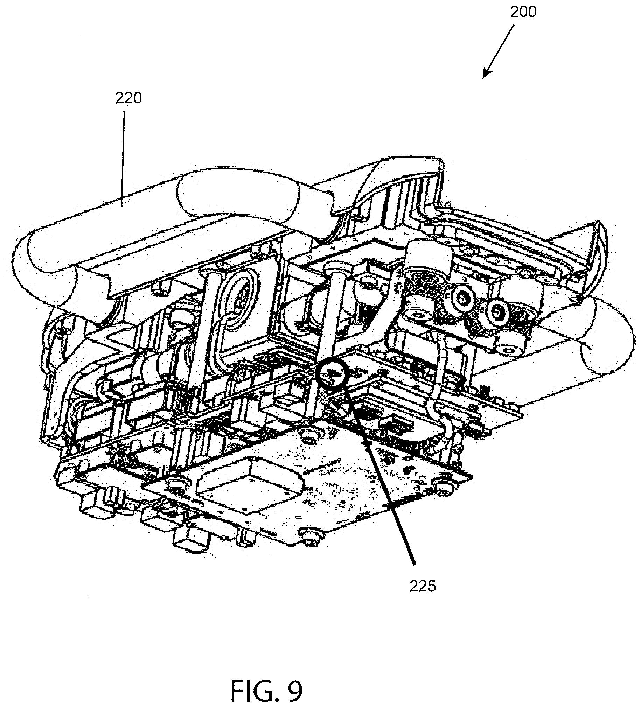

**FIG. 9** is a partial cutaway perspective front view of a feeder assembly, in accordance with the present inventive concepts.

In some embodiments, the base assembly 200 of the feeder assembly 102 can include a position sensor, such as position sensor 225 mounted to a circuit board of base assembly 200 as shown in FIG. 9. In some embodiments, the position sensor 225 can measure a relative position (e.g. orientation and/or location in 3D space) of the feeder assembly, at one or more time intervals during use, such as to determine whether feeder assembly 102 has been moved and/or to determine a geometric orientation of feeder assembly 102. Position sensor 225 can comprise a motion sensor, a displacement sensor and/or an accelerometer. In some embodiments, a multidimensional level switch, for example a bank of mercury switches, a gyroscope, or other sensor that provides angular orientation with respect to gravity may be employed for sensor 225. For purposes of the present description, the term "position sensor" is meant to include all types of sensors capable of measuring the position or displacement of an object in one or more degrees of freedom.

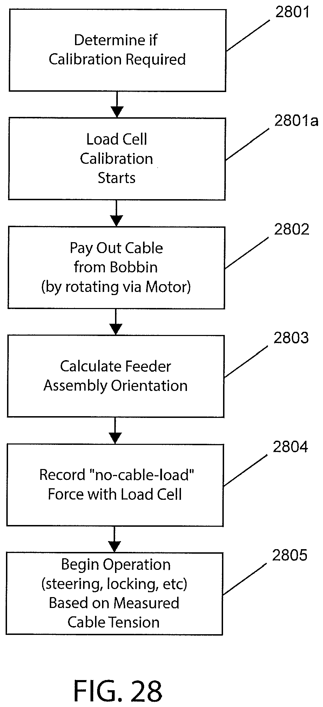

As described herein, the forces operating on the cables of probe 400 and/or the forces applied to one or more load cells 221, can change depending on the position and angular orientation of probe 400. This is also true of the forces that operate on the cables and/or the forces applied to one or more load cells 221 as a function of the position and angular orientation of other portions of feeder assembly 102. Accordingly, during a procedure, one or more calibration procedures can be performed based on the current position and angular orientation of feeder assembly 102, such as the calibration procedure described herebelow in reference to FIG. 28. Upon detection of a certain amount of feeder assembly 102 motion, as detected by the position sensor 225, the system may re-calibrate to account for variation in forces applied to the cables and/or load cell 221, as a result of the change in position of feeder assembly 102.

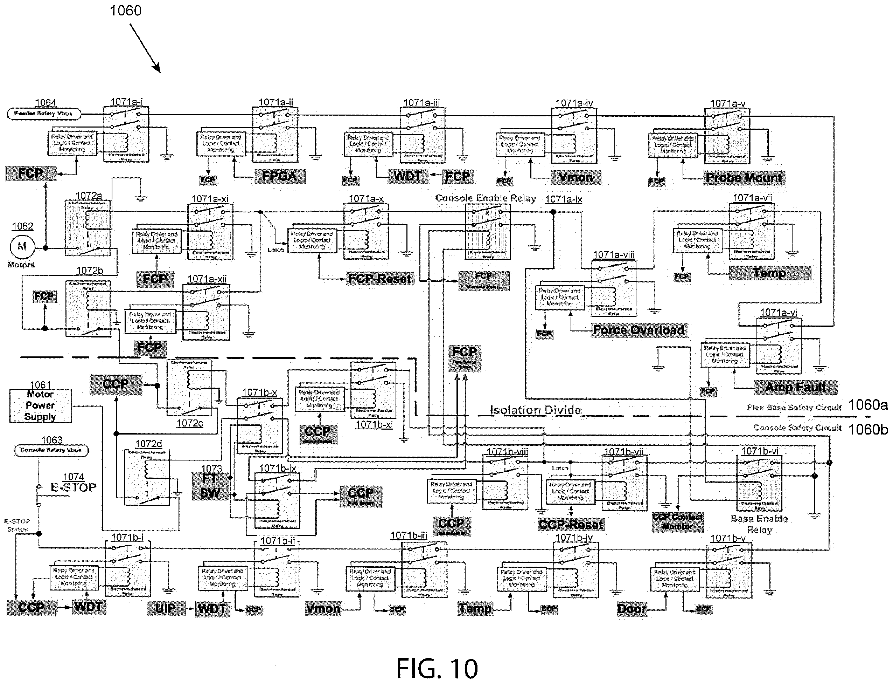

**Referring now to** **FIG. 10****,** a schematic of a safety system 1060 is illustrated, consistent with the present inventive concepts. Safety system 1060 comprises a series of switches, including safety relays 1071a-i through 1071a-v, 1071b-i through 1071b-v (generally, 1071), power relays 1072a-d (generally, 1072), and at least one user activated switch, such as foot switch 1073 and/or emergency switch 1074 (singly or collectively switch 1070). System 100 of the present inventive concepts, further comprises a power supply, motor power supply 1061, and one or more motors, motor 1062 (e.g. a cable drive motor or carriage drive motor such as motors 212 described herein). Safety system 1060 can comprise a series of mechanical, electro-mechanical or electronic relays or switches, configured to control power to one or more power relays 1072 or other electrical components of the present inventive concepts. Power relays 1072 can comprise a series of electro-mechanical or electronic relays, configured to connect and/or disconnect (herein after "control") power (e.g. power supplied from motor power supply 1061) to one or more motors (e.g. motors 1062) or other electrical components of the present inventive concepts, such as one or more motors configured to control the tension on a cable used to steer and/or lock all or a portion of articulating probe 400 and/or a motor configured to translate or otherwise drive a carriage assembly of the present inventive concepts. In some embodiments, multiple switches 1070 are connected in series, such that if any single switch 1070 is in an "open position" (such as an open switch, or an unpowered relay, such as to create an open circuit), any or all motors of the system are disconnected from the motor power supply.

Safety system 1060 further comprises a safety bus in interface unit 100b (also referred to as console 100b), console safety bus, or bus 1063. Safety system 1060 further comprises a safety bus in feeder unit 100a, feeder safety bus, 1064. In some embodiments, multiple safety relays 1071 are connected in series, such that with all safety relays 1071 in a closed position, bus 1063 and/or bus 1064 are electrically connected to one or more power relays 1072, such as one or more power relays connected in series, such that the one or more power relays 1072 are in a closed position, and motors 1062 are electrically connected to motor power supply 1061, as is described in detail herebelow.

Safety system 1060 can include one or more electronic modules, such as one or more electronic modules positioned in one or more of: top assembly 300, base assembly 200 and interface unit 100b. In some embodiments, a first safety subsystem, 1060a is positioned in the base assembly 200 and a second safety subsystem is 1060b is positioned in interface unit 100b. Safety subsystems 1060a and 1060b can be interconnected such that an open switch 1070 in either subsystem, will open one or more power relays 1072, disconnecting power from any or all motors 1062. This particular configuration can provide an advantage when system 100 includes patient electrical isolation circuitry, such as isolation circuitry positioned between interface unit 100b and feeder unit 100a.

Switches 1070 can be configured to monitor system parameters (e.g. via the control inputs to each relay 1071), such that system "fault" results in the opening of the relay 1071 configured to detect the fault which has occurred. Relays 1071, as well as switches 1073 and 1074, form a state machine that determines whether or not the motor power relays 1072 under their control can be closed based on the state of a number of inputs (e.g. all inputs relays and switches must be closed in order for power relays 1072 to close).

Safety system 1060 including each sub-system 1060a and 1060b can detect momentary drop-outs of any monitored parameter and render system 100 in a "safe state", where any or all motors 1062 are disconnected from motor power supply 1061, by opening the respective safety relay 1071 which in turn interrupts the control current to the to the power relays 1072.

Each safety relay 1071 is serially connected (e.g. arranged in a "chain" connection scheme, such as the serial connection of relays shown), and all must be closed in order for the power relays 1072 to close.

All safety relay 1071a contact statuses in the base assembly 200 are monitored by a processor in feeder unit 100a, the feeder control processor (FCP), which can be positioned in base assembly 200.

All safety relays 1071b contact statuses in the interface unit 100b are monitored by a processor within unit 100b, for example, the console control processor (CCP).

Base assembly 200 can include one or more safety relays 1071a, or other switches, as shown. The relays and/or switches can interrupt feeder safety bus 1064 when in an open position. Each relay or switch must be closed (e.g. not to interrupt bus 1064) in order to power (e.g. close) one or more power relays 1072a within base assembly 200.

Feeder Control Processor FCP controls a first safety relay 1071a-i. This relay is closed when all software checks have been passed. If software parameter monitored by FCP is outside of an acceptable range, the resulting signal will open the associated safety relay 1071a-i.

An FPGA can be include and control a safety relay 1071a-ii as shown. The FPGA closes this relay in the absence of motor encoder position or communication errors. The detection of any errors will result in the opening of the associated safety relay 1071a-ii.

A FCP Watch Dog Timer (WDT) can be included and control a safety relay 1071a-iii as shown. The FCP WDT monitors the proper performance of the FCP and must be asserted continuously (e.g. no less often than every 135ms), failure to do so (e.g. due to a software crash, FCP hardware failure or similar adverse event) will result in the WDT opening the associated safety relay 1071a-iii.

A Voltage Monitor (VMON) can be included and control a safety relay 1071a-iv as shown. The VMON circuitry monitors supply voltages on the base assembly 200, and the 15V and 28 V supplies that power electronics in base assembly 200. The critical supply voltage powering the FCP is redundantly monitored. Voltages monitored must remain at all times within a predetermined (e.g. ±10%) window of the nominal voltage otherwise a VMON error results, opening the associated safety relay 1071a-iv.

Probe Mount detection circuitry can be included and control a safety relay 1071a-v as shown. This circuitry detects the presence of the top assembly 110 of figure A-1. If top assembly 110 is not detected, the associated safety relay 1071a-v will be open.

Amplifier Fault (Amp Fault) detection circuitry can be included and control a safety relay 1071a-vi as shown. This circuitry detects proper function of an amplifier circuit. If a fault is detected, the associated safety relay 1071a-vi will open.

A Temperature Sensor (Temp) can be included and control a safety relay 1071a-vii as shown. The temperature sensor measures ambient temperature with the base and should it rise above a maximum allowable value (e.g. 60°C), the associated safety relay 1071a-vii will open.

Force Overload circuitry can be included and control a safety relay 1071a-viii as shown. This circuitry monitors the tension on any or all steering cables (e.g. steering cables used to steer and/or lock probe 112 of system 100). If the monitored tension rises above a preset maximum value, the associated safety relay 1071a-viii will open.

A Console Enable Relay 1071a-ix can be included as shown. For this relay to close, all safety relays 1071b in the console 100b, except the Base Enable Relay and CCP Reset controlled relay, and foot switch enabled relay, must be closed.

A FCP Reset Signal can be included and control a safety relay 1071a-x as shown. All preceding relays 1071a must be closed and the reset circuit must be strobed by a rising edge pulse from the FCP for this relay 1071a-x to close. The control circuitry (e.g. the circuitry which monitors the FCP Reset signal and controls the state of the associated safety relay 1071a-x is configured as a latch and the input controlled by the FCP is designed to respond only to the rising edge of the strobe signal. AC coupling is employed so that if the associated FCP port is stuck in the high state, the circuitry will not allow this relay 1071a-x to close. However, once closed the FCP can no longer open relay 1071a-x. (Relay 1071a-x is a latching relay with two inputs, one is the status of the safety circuit which must be good in order to close, and the other is a strobe pulse from the FCP. Once strobed, the relay closes and remains closed until a fault is detected elsewhere in the safety circuit.) An interruption of any of the preceding relays for a time period (typically well <10ms) will result in this relay 1071a-x opening.

Two safety relays 1071a-xi and 1072a-xii can be configured as separate enable relays which are independently controlled and monitored by the FCP, as shown. Both relays 1071a-xi and 1072a-xii must be closed in order to close the two motor power control relays 1072a and 1072b located on a Relay Daughter Board PCA located in console 100b.

Console 100b can include one or more safety relays 1071b, or other switches, as shown. The relays and/or switches can interrupt console safety bus 1063 when in an open position. Each relay or switch must be closed (e.g. not to interrupt bus 1063) in order to power (e.g. close) one or more power relays 1072b within console unit 100b.

An operator accessible emergency stop switch, E-STOP 1074, can be included as shown. The CCP monitors the status of the E-STOP switch to provide a signal correlating to an operator invoked emergency stop (e.g. a signal which can correlate to a message displayed on display 124 of FIG. 1).

A CCP Watch Dog Timer (WDT) can be included and control a safety relay 1071b-i as shown. The CCP WDT monitors the proper performance of the CCP and must be asserted continuously (e.g. no less often than every 135ms), failure to do so (e.g. due to a software crash, CCP hardware failure or similar adverse event) will result in the WDT opening the associated safety relay 1071b-i.

A User Interface Processor (UIP) WDT can be included and control a safety relay 1071b-ii as shown. The UIP WDT can monitor the proper performance of the UIP and must be asserted continuously (e.g. no less often than every 135ms), failure to do so (e.g. due to a software crash, UIP hardware failure or similar adverse event) will result in the WDT opening the associated safety relay 1071b-ii.

A Voltage Monitor (VMON) can be included and control a safety relay 1071b-ii as shown. The VMON circuitry monitors supply voltages on the Safety PCA, and the main power supply that powers electronics in the interface unit 100b. Voltages monitored must remain at all times within a predetermined (e.g. ±10%) window of the nominal voltage otherwise a VMON error results, opening the associated safety relay 1071b-ii.

A Temperature Sensor (Temp) can be included and control a safety relay 1071b-iii as shown. The temperature sensor measures ambient temperature with the interface unit 100b enclosure and should it rise above a maximum allowable value (e.g. 60°C), the associated safety relay 1071b-iii will open.

A Door Sensor can be included and control a safety relay 1071b-iv as shown. The Door Sensor is operated by a switch based safety interlock, which, if the interface unit 100b doors and/or circuit board holder are not properly in place, will result in the opening of the associated safety relay 1071b-iv.

A Base (Feeder) Enable Relay 1071b-vi can be included as shown. For this relay to close, all safety relays 1071a in the base assembly 200, except the Console Enable Relay and FCP Reset controlled relay, must be closed.

A CCP Reset Signal can be included and control a safety relay 1071b-vii as shown. All preceding relays 1071b must be closed and the reset circuit must be strobed by a rising edge pulse from the CCP for this relay 1071b-vii to close. The control circuitry (e.g. the circuitry which monitors the CCP Reset signal and controls the state of the associated safety relay 1071b-vii is configured as a latch and the input controlled by the CCP is designed to respond only to the rising edge of the strobe signal. AC coupling is employed so that if the associated CCP port is stuck in the high state, the circuitry will not allow this relay 1071b-vii to close. However, once closed the CCP can no longer open relay 1071b-vii. Relay 1071b-vii may be a latching relay with two inputs, one is the status of the safety circuit which must be good in order to close, and the other is a strobe pulse from the CCP. Once strobed, the relay closes and remains closed until a fault is detected elsewhere in the safety circuit.) An interruption of any of the preceding relays for a time period (typically well <10ms) will result in this relay 1071b-vii opening.

A Footswitch (FTSW) 1073 can be included and control a safety relay 1071b-ix as shown. Footswitch 1073 is controlled by an external footswitch. Footswitch FTSW is configured such that if the associated footswitch is not activated (e.g. depressed) by an operator, it will result in the opening of the associated safety relay 1071b-ix.

Two safety relays 1071b-x and 1071b-xi can be configured as separate enable relays which are independently controlled and monitored by the CCP, as shown. Both relays 1071b-x and 1071-xi must be closed before the FTSW can close the two console motor power control relays 1072c and 1072d located on a Relay Daughter Board PCA located in console 100b.

**FIG. 11** is a perspective illustrative view of an articulating probe system according to an embodiment of inventive concepts. **FIG. 12** is a perspective top view of base assembly 200 of the probe system of FIG. 11 in accordance with embodiments of the inventive concepts. **FIG. 13** is a bottom view of top assembly 300 of the probe system of FIG. 11 in accordance with embodiments of the inventive concepts.

As described herein, in some embodiments, for example shown at FIG. 1, feeder assembly 102 can be mounted to a feeder cart 104 at a feeder support arm 106. Feeder support arm 106 can be adjustable in height and can include a plurality of sub-arms that pivot relative to each other. Returning to FIG. 11, this adjustable configuration permits a range of orientations for positioning feeder assembly 102 relative to a patient location 608. This may include inserting elements of the probe system such as articulating probe 400 into an orifice of a patient at patient location 608. Feeder assembly 102 includes base assembly 200 and top assembly 300 that can be constructed and arranged to be removably attachable to base assembly 200.

Top assembly 300 includes articulating probe 400 for example comprising a link assembly including an inner link mechanism 420 comprising a plurality of inner links 421, and an outer link mechanism 440 comprising a plurality of outer links 441, as described in connection with various embodiments herein. For example, the inner link mechanism 420 and outer link mechanism 440 are independently advanced and retracted relative to each other in a longitudinal direction. The position, configuration (e.g. flexibility) and/or orientation of probe 400 is manipulated by a plurality of driving motors and associated cables positioned in base assembly 200 and/or top assembly 300.

In an embodiment, feeder assembly 102 can be positioned relative to feeder support arm 106 over one or more degrees of freedom at a universal joint 109. One or more supports 103 may be mounted between the base assembly 200 of the feeder assembly 102 and the feeder support arm 106, for supporting the weight of the base assembly 200 and/or feeder assembly 102 (i.e. the weight of both base assembly 200 and top assembly 300) in the region of the universal joint 109.

In some embodiments, top assembly 300 is removably attachable to the base assembly 200. In some embodiments, a hook 201 or related latch mechanism can be provided on base assembly 200 and a mating heel 301 (see FIG 13) can be provided on top assembly 300, to collectively serve as a locator joint for initially seating top assembly 300 relative to base assembly 200. Once initially seated, hook 201 and heel 301 can operate as a pivot for further seating top assembly 300 and base assembly 200. The hook 201 and heel 301 are constructed and arranged so that top assembly 300 can be pivoted in a direction opposite arrow indicator 610 until completely seated, and directly coupled to base assembly 200. At this time, handle 302 can be manually manipulated to lock top assembly 300 in position. A heel engagement assembly 230, at an interface of the hook 201 and heel 301, can be spring loaded, for example, where a spring (not shown) applies a force to the hook 201 in a direction indicated by arrow 238, to permit the hook 201 to engage with the heel 301 support mechanical play during the seating process and subsequently apply a retaining force between top assembly 300 and base assembly 200. In some embodiments, hook 201 is fixed, and not movable, and latches or couples to heel 301 when top assembly 300 is manually positioned on base assembly 200.

In some embodiments, electrical connectors 232, 332 of base assembly 200 and top assembly 300, respectively, can include mating grounding connections (e.g. mating elements holes 234 and pins 334 shown in FIGs. 12 and 13) that ensure proper grounding of top assembly 300. The electrical connectors 232, 332 can provide other electrical signal paths between the base assembly 200 and top assembly 300. Mating surfaces of the connectors 232, 332 can also be configured to accommodate the pivotal relationship of top assembly 300 relative to base assembly 200. In some embodiments, connectors 232, 332 are constructed and arranged to provide non-electrical connections, such as fluid connections (e.g. transfer of fluids such as liquids or gases and/or transfer of fluid driven force such as hydraulic or pneumatic force) or mechanical connections (e.g. connections of one or more mechanical linkages). In some embodiments, connectors 232, 332 are collectively constructed and arranged to provide a wiping force between one or more male pins prior to or during insertion into a female receptacle, such as to remove contamination from the male pins. In some embodiments, connector 232 and/or hole 234 are positioned at a floating assembly (not shown) but such as a floating circuit board which is biased in a neutral position by one or more springs that allow a position adjustment in one or more degrees of freedom during a removable connection of top assembly 300 to base assembly 200, such as to assist in connector alignment, e.g., alignment of multiple conductor electrical connections.

With reference to FIGs. 12 and 13, at the time top assembly 300 becomes completely seated on base assembly 200, capstans 216a, 216b on base assembly 200 become engaged with corresponding bobbins 316a and gears 316b on top assembly 300. In some embodiments, the mating capstans 216a and bobbins 316a can comprise cable drive capstan/bobbin pairs for driving the steering and locking cables of the inner mechanism 420 and/or outer mechanism 440 of probe 400. The capstans 216a and/or bobbins 316a are also referred to as coupling mechanisms. In some embodiments, the mating capstans 216b and gears 316b can comprise carriage drive capstan/gear pairs for driving the inner link and outer link carriages 315a, 325b, respectively, of probe 400, which in turn advance and/or retract inner links and outer links of an inner link mechanism 420 and outer link mechanism 440, respectively. Other pairings can equally apply. For example, as described above, top assembly 300 and base assembly 200 can each have electrical connectors: one male, the other female, which communicate with each other when top assembly 300 is seated on base assembly 200.

Accordingly in some embodiments, mating electrical connectors 232, 332 on the base assembly 200 and top assembly 300 engage at the time of seating. The mating electrical connectors 232, 332 serve as a pathway for electrical signals and/or other transmissions that are transferred between the base 200 and top 300 assemblies.

Once seated, feeder assembly 102 can be positioned relative to a patient location 608 for a procedure. During a procedure, an emergency such as a life-threatening situation can occur, which requires immediate removal of the probe 400 from the patient's orifice. In accordance with embodiments of the present inventive concepts, top assembly 300 can be manipulated by an operator to manually release the handle 302, whereby top assembly 300 can be pivoted in a direction up and away from the patient location 608, for example, in a direction indicated by arrow 610, using the interface of the hook 201 and heel 301 as a pivot point. This arrangement provides an element of safety, as removal of the probe 400 in this direction, i.e., away from the patient location 608 is highly desirable over removal of the probe 400 requiring top assembly 300 to be directed in a direction towards the patient location 608. At the same time, as top assembly 300 is released from base assembly 200, the capstans 216a, 216b and corresponding bobbins 316a and gears 316b become released from each other, immediately releasing the tension from all cables of probe 400. Such immediate release of cable tension is highly desirable for emergency situations, causing probe 400 to be in a limp or otherwise flexible or wiggle state, allowing quick removal of the probe 400 from the patient regardless of the geometric configuration of probe 400 prior to the release. The emergency release can be performed in various system 100 failure or non-system related emergencies, such as when power is not supplied to system 100.

Referring to FIGs. 12 and 13, to establish a quick, effective coupling and/or release of the top assembly 300 from base assembly 200 in some embodiments, top assembly 300 can include a cam 303 that is actuated by handle 302. During seating, the cam 303 can engage a corresponding cam engagement assembly 203 on base assembly 200, for locking top assembly 300 in, fixed, aligned position relative to base assembly 200. As top assembly 300 becomes fully seated, an alignment pin 204 on the base assembly engages a locator hole 304 on top assembly 300, ensuring proper alignment. In some embodiments, alignment pin 204 or locator hole 304, or both, can include tapered upper surfaces to accommodate mechanical play to assist in the alignment process. It should be appreciated that one or more alignment pins in bottom assembly 200 can be replaced with receiving holes, where the one or more mating holes of top assembly 300 are each accordingly replaced with an alignment pin configured to mate with the receiving hole of bottom assembly 200.

In some embodiments, a set of alignment pins, and/or pins 205 and corresponding location holes 305 can further be included for positioning a sterile drape between top assembly 300 and base assembly 200. In some embodiments, top assembly 300 including the probe 400 is a sterile apparatus that comes in contact with the patient, while the base assembly 200 and feeder arm support 106 and feeder cart 104 are not sterile. For this reason, a sterile drape can be applied between top assembly 300 and base assembly 200 so that base assembly 200 can be reused for subsequent procedures. The mating pins 205 and location holes 305 communicate with similarly positioned apertures on the drape for ensuring proper positioning of the drape during a procedure.

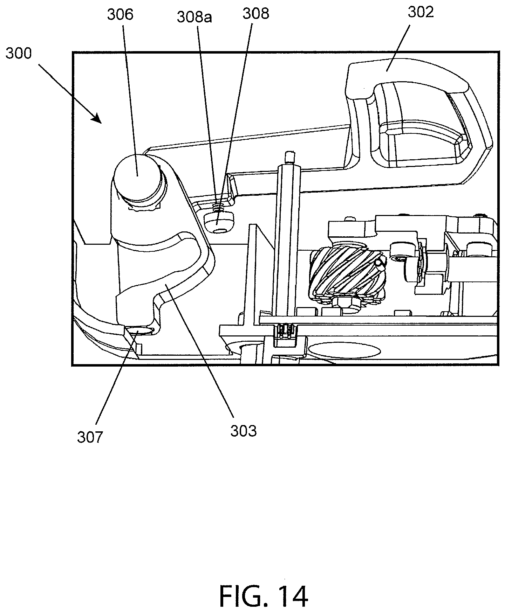

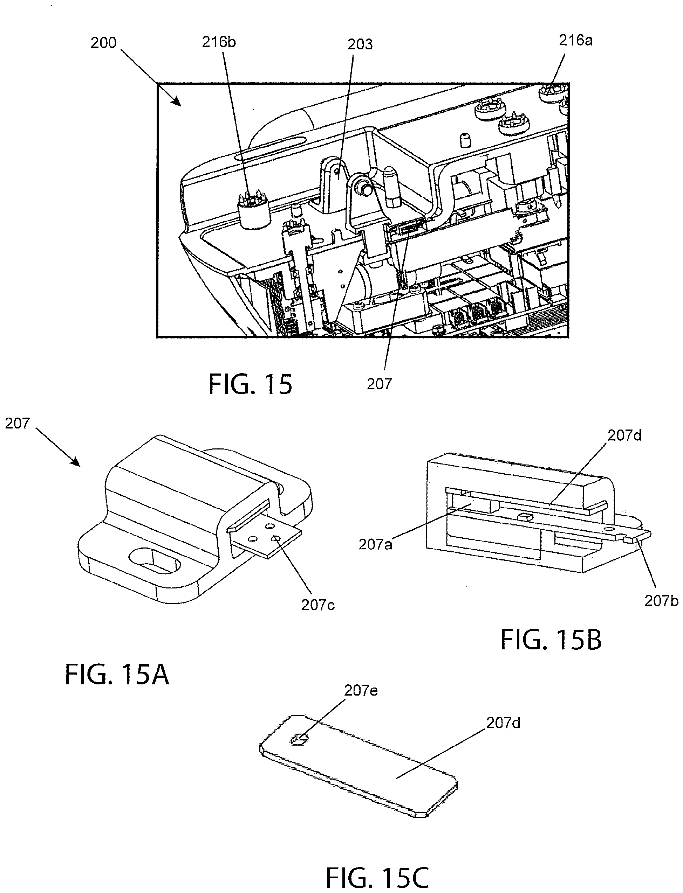

**FIG. 14** is a perspective cutaway view of a handle 302 of a top assembly 300 of a feeder assembly 102 of an articulating probe system 100, according to an embodiment of inventive concepts. **FIG. 15** is a perspective cutaway view of a base assembly 200 of a feeder assembly 102 of an articulating probe system 100 according to an embodiment of inventive concepts. **FIGs. 15A-15C** are perspective views of proximity sensor componentry, in accordance with embodiments of inventive concepts.

Referring to **FIG. 14****,** in some embodiments, top assembly 300 can include handle 302 that pivots at pivot 306 to engage cam 303 to the cam engagement assembly 203 of base assembly 200. In some embodiments, a portion of the cam 303 can include a magnet 307 having a magnetic field of sufficient strength for emitting the magnetic field into base assembly 200.

Referring to **FIG. 15****,** base assembly 200 can include a proximity sensor 207 suitable for detecting the magnetic field emitted by the magnet 307 (FIG. 14) of top assembly 300, such as a magnet 307 positioned in a portion of handle 302. Accordingly, proximity sensor 207 is positioned in the vicinity of the region where magnet 307 of handle 302 is positioned when top assembly 300 is properly seated and locked into position on the base assembly 200.

In some embodiments, a bumper 308 can be located on the handle 302 to provide for tactile feedback to an operator when engaged. The bumper 308 can comprise a rubber or soft plastic material that is slightly deformable. In some embodiments, the bumper can have a threaded base 308a as shown, so that its vertical position, relative to the handle 302 can be adjustable (e.g. to adjust the amount of tactile feedback received). In alternative embodiments, the bumper 308 can instead be positioned at an upper surface of the base assembly 200 to contact handle 302 as handle 302 is moved to a seated position.

Referring to **FIGs. 15A-15C****,** proximity sensor 207 can comprise, in some embodiments, a magnetic sensor, for example, a Hall-effect sensor 207a, seated on an electrical board 207b having electrical contacts 207c for transferring electrical signals to and from sensor 207. In some embodiments, more accurate positioning is required than available by the Hall sensor, and accordingly a Mu-metal plate 207d can be included. Mu-metal plate 207d blocks all magnetic field transfer to the Hall-effect sensor. An aperture 207e within in plate 207d as shown allows magnetic fields from magnet 307 to pass (e.g. when top assembly 300 is properly engaged with base assembly 200), effectively increasing the positioning sensitivity of the proximity sensor 207. In some embodiments, the Mu-metal plate 207d can have two apertures 207e, one at each end, so that the plate 207d is thereby symmetric (e.g. to allow placement in manufacturing in either direction, and potentially with either side oriented up). Such an embodiment would ease manufacturing constraints, eliminating the possibility of erroneous insertion of the plate 207d.

Although the illustrative embodiments depict the magnet 307 positioned on top assembly 300 and the proximity sensor 207 positioned on base assembly 200, in other embodiments, their positioning can be reversed; namely, the magnet 307 can be positioned on base assembly 200 and the proximity sensor 207 positioned on top assembly 300. Further, although the above embodiments depict magnet 307 and sensor 207 positioned in a region of the cam 303 and cam engagement assembly 203, their placement in other regions of top assembly 300 and base assembly 200 are also applicable to the inventive concepts.

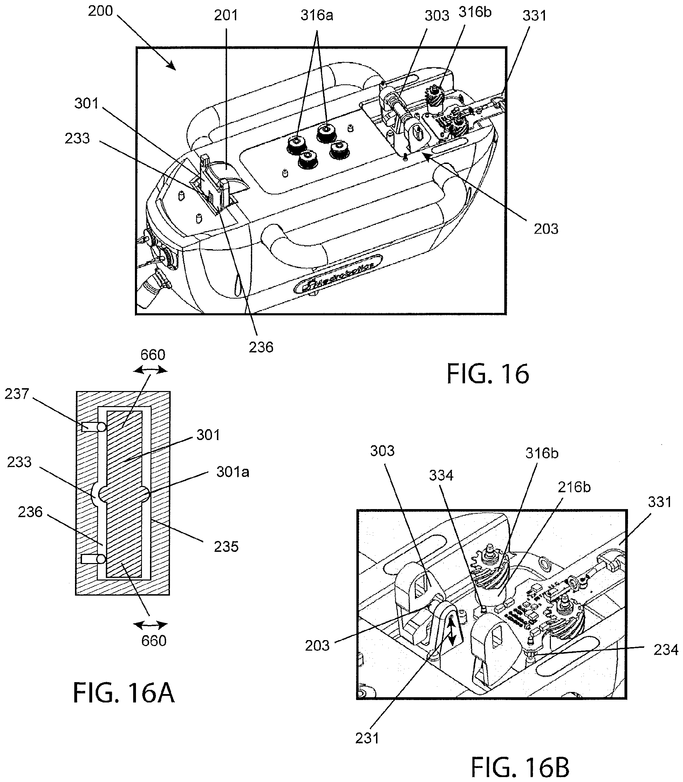

FIG. 16 is a perspective partial cutaway view of a base assembly 200 of a feeder assembly 102 of an articulating probe system 100 according to an embodiment of inventive concepts. FIG. 16A is a section view of a base assembly 200 and of the interaction of the heel 301 and base cutout 233 according to an embodiment of inventive concepts. FIG. 16B is a closeup perspective view of the cam engagement assembly 203 of the base, in accordance with embodiments of inventive concepts.

**Referring to** **FIGs. 16 and 16B****,** a partial cutaway view of the base assembly 200 is shown, along with certain components of top assembly 300 engaged with corresponding components of base assembly 200, including the heel 301, bobbins 316a, carriage gears 316b, cam 303, and electronics module 331 of top assembly 300. Capstans 216a of base assembly 200 are engaged with bobbins 316a but hidden from view in FIG. 16. Capstans 216b of base assembly 200 are engaged with carriage gears 316b but also hidden from view in FIG. 16 (shown in FIG. 16b). It is assumed that top assembly 300 is properly mounted and secured to the base assembly 200. Referring to FIG. 16B it can be seen that the cam 303 mates with the cam engagement assembly 203 when top assembly 300 is properly installed. As described herein the cam engagement assembly 203 can be spring-biased in a vertical direction indicated by arrow 231 to allow for mechanical play in the seating and securing process. Alignment pins 334 of the top assembly 300 mate with corresponding holes 234 of bottom assembly 200 to ensure proper electrical connectivity between the base assembly connector 232 and top assembly 300 connector 332 (see FIGs. 12 and 13).

Referring again to FIG. 16A, it can be seen that the heel 301 of top assembly 300 is engaged with the hook 201 of base assembly 200. In some embodiments, the interaction of the heel 301 and hook 201 can be the first point of contact in the seating process of top assembly 300 relative to base assembly 200. As described herein, the heel 301 / hook 201 interface can provide the pivot point of top assembly 300 during seating and release, and serve as an emergency release feature, by providing pivot of top assembly 300 "up and away" from the patient, as described herein, instead of in a direction toward the patient. In some embodiments, the hook 201 and/or heel 301can be spring-loaded to allow for mechanical play in the seating and securing process.

In some embodiments, the heel 301 can include a ridge feature 301a at its center portion. The ridge feature 301a can operate as a contact point with a corresponding datum plate 235 surface of the receiving slot 236 of base assembly 200. This configuration longitudinally aligns top assembly 300 with base assembly 200 while allowing for a minimum, predetermined amount of angular offset in their positioning, for example, in a direction of rotation indicated by arrows 660. Such play in angular offset accommodates the alignment process during seating of top assembly 300 relative to base assembly 200. Ball plungers 237 may be included in the receiving slot 236 opposite the datum plate 235 to maintain or bias the heel 301 against the datum plate 235, also referred to as a registration plate at base assembly 200.

As described herein, during an emergency release of top assembly 300 and probe 400 relative to base assembly 200, the handle 302 can be lifted, such that top assembly 300 is then free to rotate about the hook 201 of base assembly 200. As described herein, top assembly 300 rotates in a direction indicated by arrow 610 of FIG. 11, up and away from the patient location 608. As top assembly 300 pivots, bobbins 516a and gears 516b are separated from, or otherwise removed from or lifted off, the capstans 216a, 216b, respectively. This, in turn, releases tensions in all cables of probe 400, allowing safe removal of probe 400 from the patient, as the probe becomes "limp" and/or at least malleable. At the same time, upon pivoting, magnet 307 is no longer detected by the proximity sensor 207, so electronic subsystems, sensors, and so on of system 100 can become aware of the release. Alignment pins 205, 334 become disengaged form their corresponding holes 305, 234. Electronics become disengaged at connectors 232, 332, cutting power the system camera and/or other systems electronics.

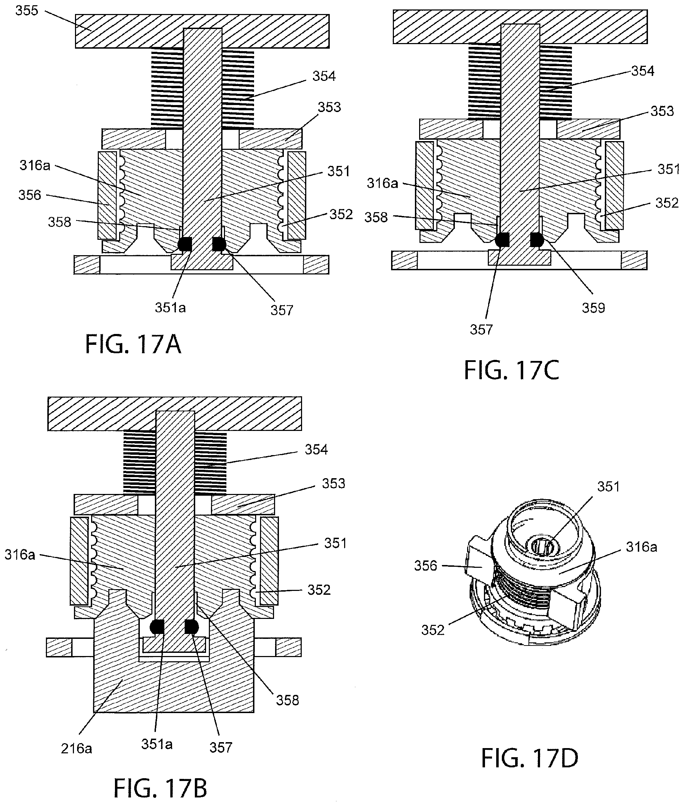

**FIG.17A** is a side view of a cable bobbin of the top assembly in a shipping condition according to an embodiment of inventive concepts. **FIG. 17B** is a side view of a cable bobbin of the top assembly in an operating condition according to an embodiment of inventive concepts. **FIG. 17C** is a side view of a cable bobbin of the top assembly in a release condition according to an embodiment of inventive concepts. **FIG. 17D** is a perspective view of a cable bobbin of the top assembly including a cable retention clip according to an embodiment of inventive concepts.

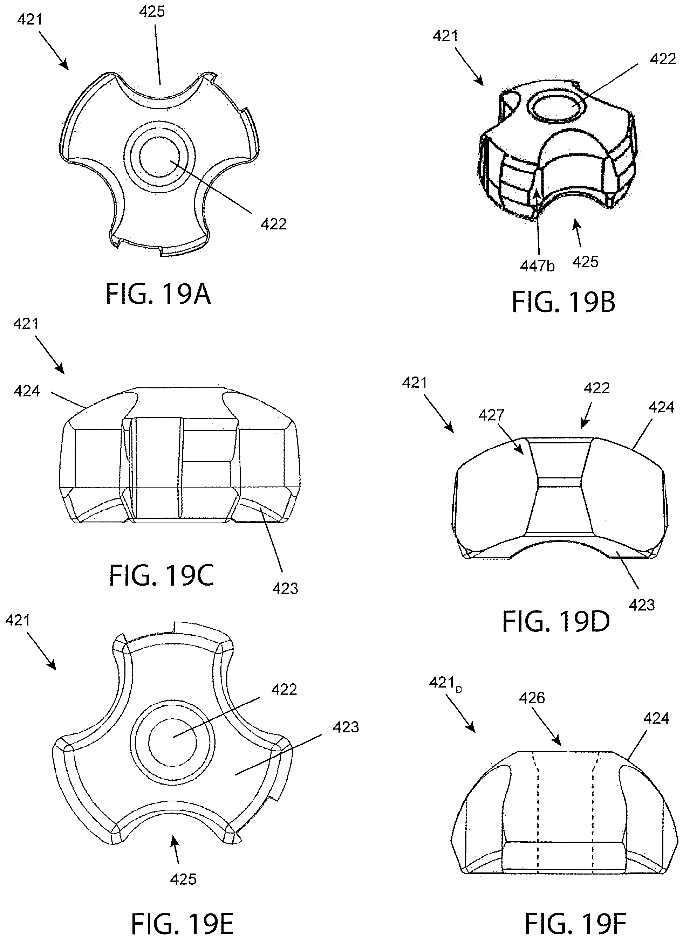

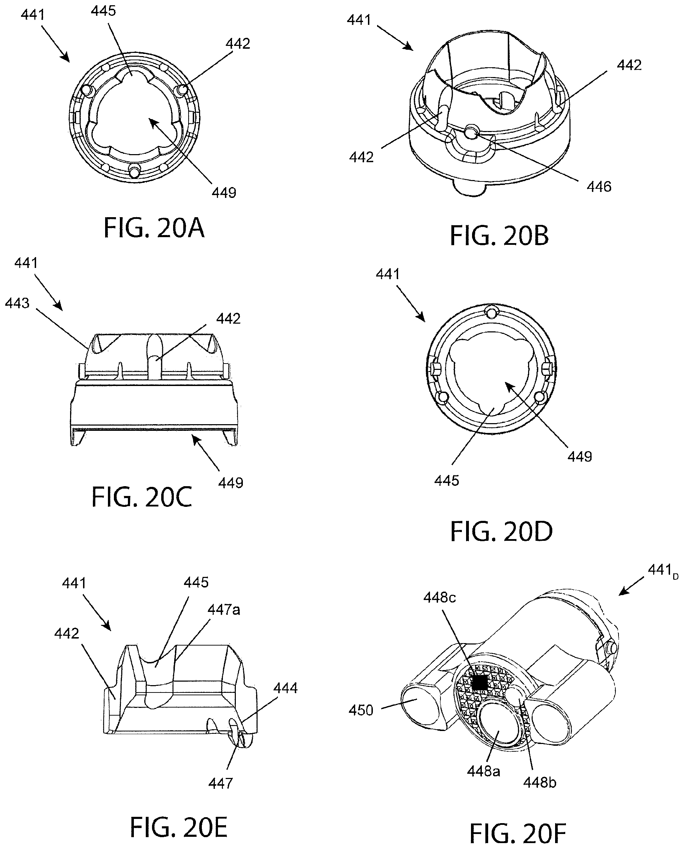

**Referring to** **FIG. 17A****,** a cable bobbin 316a is constructed and arranged to be centered about, and rotate about, a bobbin axle 351. The cable bobbin 316a includes cable grooves 352 for receiving a cable, for example, a steering cable 350 shown in FIG. 5B. In some embodiments, the cable can comprise a steering and locking cable which steers and/or reversibly tightens to lock or stiffen the outer link mechanism 440 and/or the inner link mechanism 420 described herein. The cable grooves 352 can be a single groove formed in a helical pattern about the cylindrical outer surface of the bobbin 316a in which at least a portion of a steering cable 350 can be positioned. In some embodiments, the cable bobbin 316a is seated on a bobbin washer 353 in turn interfacing with a bobbin spring 354 so that the washer 353 is positioned between the spring 354 and the bobbin 316a. The bobbin spring 354 may be seated in a bobbin plate 355, such as at least partially positioned in a recess of bobbin plate 355 (recess not shown).Bobbin spring 354 is positioned between the bobbin plate 355 and the washer 353, and allows for vertical travel of the bobbin 316a relative to the bobbin plate 355. Here, an axle is fixed to plate 355 and the bobbin 316a rotates about the axle, and can travel vertically on the axle (e.g. against the force of the spring 354, such as when engaged with capstan 216a. In some embodiments, during manufacture, a first end of a cable is coupled to a distal link of the probe 400, for example, a distal inner link 421_{D} (shown in FIG. 19F) or distal outer link 441_{D} (shown in FIG. 20F) and a second end is wound about and secured to a bobbin 316a, such that tension is maintained in the cable between the distal link and the bobbin.. During shipping, it is desired that the cables not lose tension or become released. "Free" unspooling or other loosening of cables can create an unreliable state of the inner or outer probe and these control cables. Once manufactured, the cables remain under tension at all times to prevent unspooling or other loss of tension that can result in an undesired, unknown and/or unrecoverable state of the probe.

In some embodiments, to prevent release of the cable from cable grooves 352, a cable clip can be included, such as clip 356 shown (e.g. in the perspective view of Fig. 17D), which rotatably engages bobbin 316a allowing cable to be collected onto bobbin 316a and paid out or extended from bobbin 316a while maintaining the portion of the cable surrounding bobbin 316a or otherwise positioned in the cable grooves 352 helically wound about the bobbin 316a in close proximity to bobbin 316a.

In some embodiments, to prevent release of the cable from the cable grooves 352 and/or to otherwise prevent de-tensioning (e.g. unwinding) of the cable prior to attachment of a top assembly 300 to a base assembly 200 (e.g. during shipment of one or more top assemblies 300 to a clinical or other operator site), an o-ring 357 can be fixedly attached or otherwise seated about a neck region of the bobbin axle 351, such as in groove 351a of axle 351 as shown. In this embodiment, the bobbin 316a can be provided with a counter bore 358 of an inner diameter slightly less than an outer diameter of the o-ring 357 so that the o-ring 357 can be positioned in the counter bore 358 and directly about the periphery of the wall of the bobbin 316a forming the counter bore 358 with sufficient force to prevent rotation of the bobbin 316a. The frictional relationship between the o-ring 357 and the counter-bore 358 operates to resist rotation of the bobbin 316a, or otherwise hold the bobbin 316a in a stationary position, and therefore resist de-tensioning of the cables prior to attachment of top assembly 300 to base assembly 200 (e.g. during shipment of one or top assemblies 300). The force of the spring 354 operating on the washer 353 maintains the o-ring 357 in the counter bore 358 until top assembly 300 is ready to be attached to a base assembly 200 to perform a clinical or other procedure.

**Referring to** **FIG. 17B****,** after a top assembly 300 is attached to a base assembly 200 (e.g. during a clinical procedure), a capstan 216a of the base assembly 200 mates with a corresponding bobbin 316a, and in doing so, pushes the bobbin 316a in an upward direction, compressing the spring 354 and removing a frictional engagement between o-ring 357 and bobbin 316a by separating the o-ring 357 from the counter bore 358. As a result, the bobbin 316a operates in response to its corresponding capstan 216a and capstan drive assembly, without frictional resistance being applied to bobbin 316a, since o-ring 357 is no longer in frictional engagement with bobbin 316a.

**Referring to** **FIG. 17C****,** after a release of top assembly 300 from base assembly 200 (e.g. after procedure completion or after an emergency release), the capstan 216a is no longer in contact with the bobbin 316a. Accordingly, the spring 354 operates to apply a force that pushes the bobbin washer 353 and bobbin 316a in a downward direction as shown. The o-ring 357 once again engages an upper surface of the counter bore 358, providing a slight, but not full, resistance to bobbin 316a movement. A chamfer 359 may be included on the exit of counter bore 358 as shown, such that when o-ring 357 is biased against chamfer 359 by spring 354 (as shown in FIG. 17C and resulting after top assembly 300 is removed from base assembly 200), some (minimal) frictional engagement between bobbin 316a and o-ring 357 is present (but less than that which occurs in the configuration of FIG. 17A).

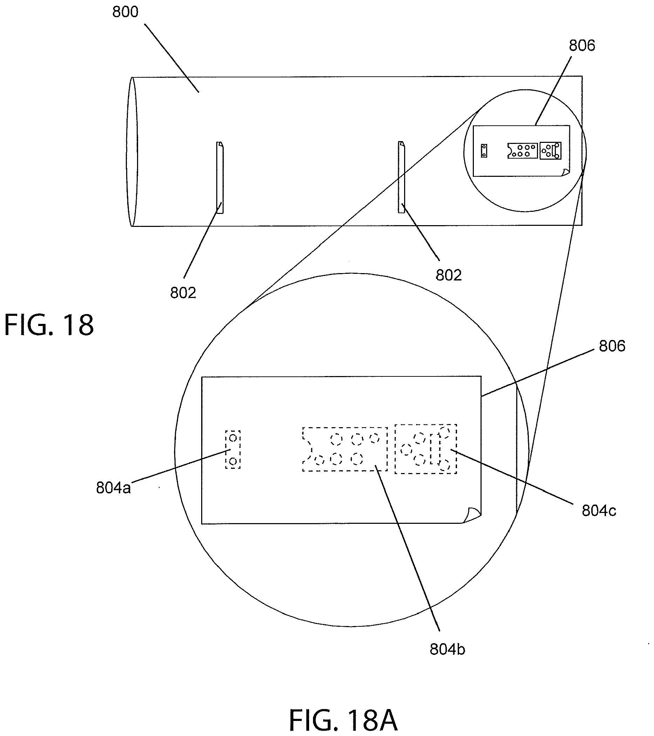

**FIG. 18** is a top view of a sterile drape assembly according to an embodiment of inventive concepts. FIG. 18A is a magnified view of a portion of the drape assembly of FIG. 18. In some embodiments, the sterile drape can comprise HDPE or other flexible, sterilizable material. As described herein, a sterile drape 800 is provided during a procedure, to maintain sterility in the sterile environment, and to shield non-sterile portions of the system, and to separate reusable components of an articulating probe assembly from its sterilized, but single use, components. One or more alignment plates 804, such as alignment plates 804a, 804b and 804c shown, are provided to align the pass-through regions of the base assembly 200 and top assembly 300 of the feeder assembly 102. Alignment plates 804a, 804b, 804c include the pass-through regions (e.g. openings through which one or more components of top assembly 300 and/or base assembly 200 can pass). Straps 802 may be provided for attaching the drape 800 to features of the system console and feeder arm.

In preparation for a procedure, it is desired that the sterile drape be applied about the base assembly 200. After this, a certain amount of time may pass before top assembly 300 is mounted to the base assembly 200. During this time, maintenance of sterility is desired.

Accordingly, embodiments of the present inventive concepts provide a removable plate cover 806 that covers the region of the alignment plates 804. The removable plate cover 806 can be removed just prior to attachment of the top assembly 300 to the base assembly 200. In some embodiments, the removable plate cover can cover the pre-formed openings in the alignment plates 804. In some embodiments, the removable plate cover 806 can be bonded to the alignment plate 804 and/or surface of the drape 800, and peeled therefrom by a technician or other operator just prior to use.

**FIGs. 19A-19F** illustrate various views of embodiments of an inner link 421 of the present inventive concepts. In particular, FIG. 19A is a top view of the inner link 421, FIG. 19B is a perspective view of the inner link 421, FIG. 19C is a side view of the inner link 421, FIG. 19D is a side-sectional view of the inner link 421, and FIG. 19E is a bottom view of the inner link 421. FIG. 19F is a side view of a distal inner link 421_{D}, in accordance with an embodiment of the present inventive concepts.

**FIGs. 20A-20F** illustrate various views of embodiments of an outer link 441 of the present inventive concepts. In particular, FIG. 20A is a top view of the outer link 441, FIG. 20B is a perspective view of the outer link 441, FIG. 20C is a side view of the outer link 441, FIG. 20D is a bottom view of the outer link 441, and FIG. 20E is a side-sectional view of the outer link 441. FIG. 20F is a perspective view of a distal outer link 441_{D}, in accordance with an embodiment of the present inventive concepts.

Inner links 421 of FIGs. 19A-19F and outer links 441 of FIGs. 20A-20F can comprise the same, similar, or dissimilar materials, such as is described in detail herebelow. In some embodiments, inner links 421 and/or outer links 441 are constructed and arranged similar to the inner and outer links described in applicant's co-pending U.S. Patent Application Serial No. 13/880,525, filed April 19, 2013 and/or U.S. Patent Application Serial No. 14/343,915, filed September 12, 2012, the contents of each of which is incorporated herein by reference in its entirety.

In some embodiments, articulating probe 400 of the present inventive concepts comprises an inner link mechanism 420 including between 10 and 300 inner links 421. In some embodiments, the inner link mechanism 420 can include between 50 and 150 inner links 421. In some embodiments, the inner link mechanism 420 can include between 75 and 95 inner links 421, such as approximately 84 inner links 421. In some embodiments, inner links 421 comprise a length between 0.05" and 1.0" In some embodiments, the length of an inner link 421 can range between 0.1" and 0.5", such as approximately 0.2".

In some embodiments, inner links 421 comprise an effective outer diameter of between 0.1" and 1.0". In some embodiments, an inner link 421 can include an effective outer diameter of between 0.2" and 0.8", such as an effective outer diameter of approximately 0.35".

In some embodiments, inner links 421 comprise a lumen, or channel 422, configured to slidingly receive a cable to control locking. Channel 422 can be centered in the relative geometric center of inner links 421, and can comprise a diameter between 0.01" and 0.9", such as a diameter between 0.02" and 0.3", such as a channel with a minimum diameter of approximately 0.07" (e.g. a minimum diameter of a channel 422 with a tapered or hour-glass shaped profile as shown and described herein). In some embodiments, one or more inner links 421 comprise multiple lumens, such as to slidingly receive a cable in each lumen. One or more cables extending through lumens of the inner links 421 in this manner may allow both locking and steering of the inner link mechanism 421 of probe 400, for example, in a manner described herein.

In some embodiments, inner links 421 comprise one or more materials configured to optimize locking of inner links 421 relative to each other. In some embodiments, inner links 421 comprise a high-friction material, such as an injection-molded or other material comprising glass fibers or the like. In some embodiments, inner links 421 comprise an isotropic construction, or at least one or more isotropic portions. In some embodiments, inner links 421 comprise a plastic material such as Noryl™ material or the like.

Inner links 421 shown in FIGs. 19A-19F can each comprise a proximal surface 423 with a spherical geometry and/or a distal surface 424 with a spherical geometry. In some embodiments, both proximal surface 423 and distal surface 424 comprise a spherical geometry, such as to create a spherical surface to spherical surface interface between adjacent inner links 421 that maximizes locking (e.g. by increasing surface contact between adjacent inner links 421). In some embodiments, inner link 421 proximal surface 423 comprises a similar radius of curvature to distal surface 424. In some embodiments, inner link 421 proximal surface 423 comprises a radius of curvature of between 0.1" to 1.0". In some embodiments, a proximal surface 423 of an inner link can include a radius of between 0.3" and 0.7", such as a radius of approximately 0.55". In some embodiments, inner link 421 distal surface 424 comprises a radius of curvature of between 0.1" to 1.0". In some embodiments, a distal surface 424 of an inner link 421 can include a radius of between 0.3" and 0.7", such as a radius of approximately 0.55".

In some embodiments, inner links 421 comprise one or more working channel recesses, such as the three recesses 425 shown. Inner link 421 recesses 425 align with outer link 441 recesses 445 described herein. Recesses 425 can comprise a geometry constructed and arranged to receive a tool with a diameter between 1.0mm and 10.0mm, such as a diameter between 2.0mm and 5.0mm, or a diameter of approximately 2.5mm (e.g. corresponding to a recess 425 diameter of approximately 3.3mm). Details regarding various recess geometries in accordance with some embodiments are described herein.

In some embodiments, the outermost inner link 421, or inner link 421 most distal in the inner link mechanism 420 comprises a different geometry than the more proximal inner links, such as distal inner link 421_{D}, whose side view is illustrated in FIG. 19F. Distal inner link 421_{D} can comprise a different geometry than the other inner links 421, such as a bullet-nose geometry shown in FIG. 19F. Distal inner link 421_{D} can comprise an opening 426 (e.g. a spherical shelf or other tapered opening) configured to receive an anchoring member (not shown but such as a ferrule) positioned on the distal end of a cable inserted through the series of inner links 421. Distal inner link 421_{D} can comprise a larger taper (e.g. less blunt) on its distal surface 424 than the distal surfaces of other inner links 421, such as to provide a sufficiently tapered distal end of inner link mechanism 420, such as to ease advancement of inner link mechanism 420 within an interior region of outer link mechanism 440. In some embodiments, distal inner link 421_{D} comprises a different (e.g. stronger) material than other inner links 421, such as a metal, stainless steel or aluminum, for example to prevent damage to distal inner link 421_{D} at opening 426 due to forces exerted by anchoring the cable.

Referring to FIGs. 20A-20F, in some embodiments, articulating probe 400 of the present inventive concepts comprises an outer link mechanism 440, that may include between 5 and 150 outer links 441. In some embodiments, the outer link mechanism 440 can include between 10 and 100 outer links 441. In some embodiments, the outer link mechanism 440 can include between 20 and 80 outer links 441, such as approximately 56 outer links 441. In some embodiments, articulating probe 400 comprises more inner links 421 than outer links 441, such as at least 10% more inner links 421, such as at least 50%, 100%, 200%, 300% or 500% more inner links 421. The larger proportion of inner links 421 can correlate to a shorter relative length of inner link 421 which can reduce binding or other translation issues that otherwise might be encountered during advancement and/or retraction of inner link mechanism 420 within at least a portion of outer link mechanism 440. In some embodiments, outer links 441 comprise a length between 0.1" and 2.0", such as between 0.2" and 1.0", such as approximately 0.4".

In some embodiments, outer links 441 comprise an effective outer diameter of between 0.2" and 2.0", such as an effective outer diameter of between 0.4" and 1.6", such as an effective outer diameter of approximately 0.68".

In some embodiments, outer links 441 comprise two or more lumens, such as the three channels 442 shown. One or more cables may extend through the channels 442 of the outer links 441. For example, the channels 442 may each configured to slidingly receive a cable to control both locking and steering of outer link mechanism 440. In some embodiments, channels 442 can be positioned with equal circumferential spacing (e.g. the approximately 120° spacing shown) within outer links 441. In some embodiments, a channel 442 can comprise a diameter between 0.06" and 0.4". In some embodiments, a channel 442 can comprise a diameter between 0.01" and 0.2". In some embodiments, a channel 442 may have a minimum diameter of approximately 0.047" (e.g. a minimum diameter of a channel 442 with a tapered or hour-glass shaped profile as shown and described herein).

In some embodiments, one or more outer links 441 comprise an inner link channel 449 in a center region of the outer link 441, which extends along a longitudinal axis of the outer link 441. One or more inner links 421 of the inner link mechanism 420 can be positioned in the inner link channels 449 of the outer links 441, and can translate (e.g. advance or retract) relative to the inner link channels 449 of the outer links 441.

In some embodiments, outer links 441 comprise one or more materials configured to optimize both locking and steering of outer links 441 relative to each other. In some embodiments, a set of two or more outer links 441 positioned in a distal portion of outer link mechanism 440 comprise different materials (e.g. more lubricious materials configured to improve steering) than the materials used in two or more outer links 441 positioned in a proximal portion of outer link mechanism 440. In some embodiments, between 2 and 10 (e.g. between 2 and 7) outer links 441 positioned in a distal portion of outer link mechanism 440 comprise a more lubricious material than outer links 441 positioned in a more proximal portion of outer link mechanism 440, such as when the articulating probe 400 of the present inventive concepts is constructed and arranged to steer between 2 and 10 (e.g. between 2 and 7) outer links 441 simultaneously (e.g. an operator determined number of outer links 441 selected for steering). In some embodiments, the more lubricous material comprises one or more of: Ultem material; Ultem EFL 36 or similar material; Ultem 1000 or similar material; a Teflon additive; a material selected for enhanced rigidity of outer link 441; a material selected for minimal compression of outer link 441; and combinations of these. In some embodiments, the most distal outer link 441 comprises Ultem 1000 or similar material. In some embodiments, the less lubricious material of the more proximal outer links 441 comprises a material selected from the group consisting of: a liquid crystal polymer; IXEF or similar material; Noryl or similar material; and combinations of these. In some embodiments, the geometry and/or material of the more proximal outer links 441 is configured to lock outer link mechanism 440, and the geometry and/or material of the more distal outer links 441 is configured to both lock and steer outer link mechanism 440.

In some embodiments, one or more outer links 441 comprise a glass fiber material, such as an outer link 441 which includes approximately 30% glass fiber fill. In some embodiments, the most distal outer link 441D does not comprise, or is otherwise absent, a glass fiber fill, or comprises less fiber fill relative to other outer links 441

In some embodiments, one or more outer links 441 (e.g. the most distal outer link 441_{D}) comprise an opaque material, such as to prevent light from passing through the outer surface of one or more portions of outer link mechanism 440. Additionally or alternatively, one or more outer links 441 can comprise a matte and/or dark finish, such as to prevent or minimize glare off of the outer surface of one or more portions of outer link mechanism 440.

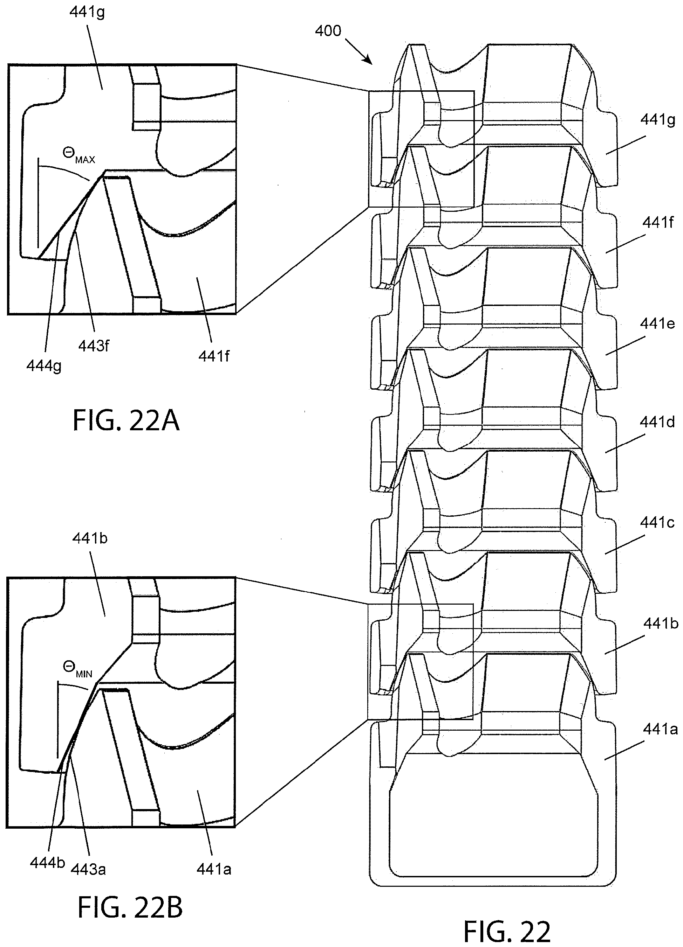

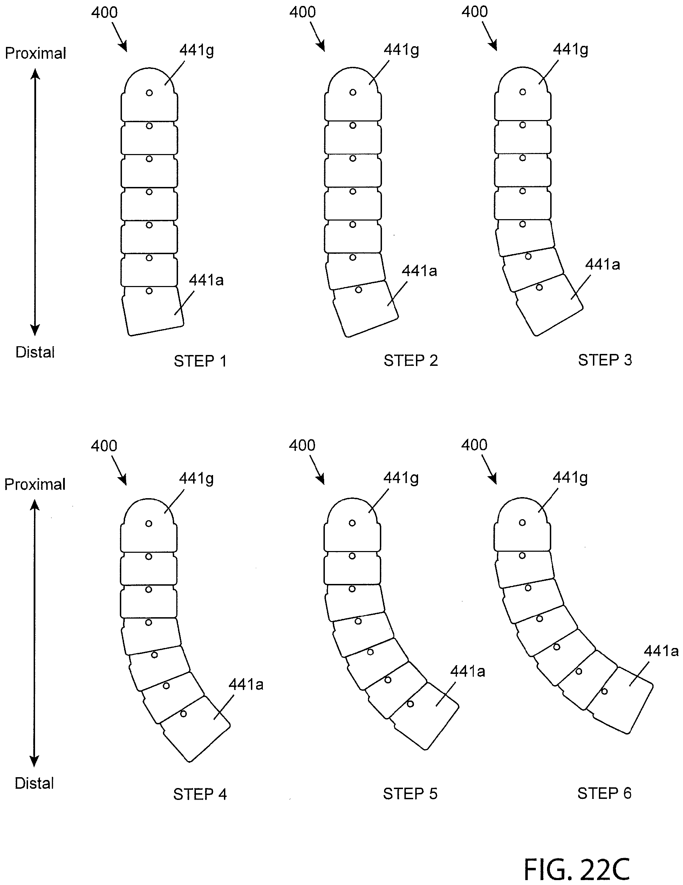

In some embodiments, the series of outer links 441 in a distal portion of outer link mechanism 440 are configured to articulate (e.g during steering) in a cascading order (e.g. from distal to proximal), such as is described in detail in reference to FIG. 22 herebelow.

One or more outer links 441 can each comprise a proximal surface 443 with a spherical geometry (shown) and/or a conical geometry. In some embodiments, distal surface 444 comprises a dissimilar geometry, such as a conical geometry (shown), such as to create a conical surface to spherical surface interface between adjacent outer links 441 that enhances steering (e.g. by reducing surface contact between adjacent outer links 441 in a manner to reduce sticking). Alternatively, distal surface 444 of an outer link 441 can comprise a similar geometry as that of proximal surface 443 of an adjacent outer link 441 positioned for directly abutting the distal surface 444 of the outer link 441 (shown in FIG. 22B). for example, the distal surface 444 of the outer link 441 a spherical geometry similar to a spherical geometry of proximal surface 443 of adjacent outer link 441. In some embodiments, outer link 441 proximal surface 443 comprises a radius of curvature of between 0.1" to 1.0", such as a radius of between 0.3" and 0.8", such as approximately 0.57". In some embodiments, outer link 441 distal surface 444 comprises a cone with a taper between 5° to 70°,. In some embodiments, the cone of the distal surface 444 of the outer link 441 has a taper between 10° and 65°, such as a taper of approximately 23°.

Although the outer links 441 are described herein as having a distal surface 444 and a proximal surface 443, the use of "proximal" and "distal" in this form is for the purpose of discussion only. The relative positions of the surfaces 443, 444 of each link can be proximal or distal relative to the overall assembly of the outer link mechanism 440, depending on the configuration. The use of the terms "proximal" and "distal" are not used herein in a limiting manner to imply that the positions of the surfaces 443, 444 are at proximal or distal locations relative to the proximal and distal ends of the overall assembly of the outer link mechanism 440.

In some embodiments, outer links 441 comprise one or more working channel recesses, such as the three recesses 445 shown in FIGs. 20A-22. In some embodiments, outer link 441 recesses 445 align with inner link 421 recesses 425, for example, described herein. Recesses 445 can comprise a geometry constructed and arranged to receive a tool with a diameter between 1.0mm and 10.0mm. In some embodiments, recesses 445 have a diameter between 2.0mm and 5.0mm, or a diameter of approximately 2.5mm (e.g. corresponding to a recess 445 diameter of approximately 3.3mm). The working channel recesses 445 and 425 of the outer links 441 and inner links 421, respectively, are configured to accommodate the translation of tools within them at all potential configurations of articulating probe 400. For example, the geometry of the recesses 445 ,425 are configured to accommodate all potential minimum and maximum radius of curvatures for the multiple curved segments of inner link mechanism 420 and outer link mechanism 440.

In some embodiments, two or more outer links 441 comprise anti-rotation elements, such as pin 446 and slot 447 shown. The anti-rotation elements can be constructed and arranged to prevent one or more of the following events (e.g. during steering and/or during translation of the inner link mechanism 420 or the outer link mechanism 440); changes in working channel shape; pinching of tools or filaments passing through a working channel; moving of tools or filaments passing through a working channel; pinching of cables passing through channels 422 and/or 442, respectively; pinching or binding of inner link mechanism 420 as inner link mechanism 420 translates (e.g. advances or retracts) relative to outer link mechanism 440; and combinations of these. In some embodiments, pin 446 and slot 447 are constructed and arranged as described in applicant's co-pending United States Patent Application Serial No. 14/343,915, filed September 12, 2013, the content of which is incorporated herein by reference in its entirety.

In some embodiments, the most distal outer link comprises a different geometry than the more proximal outer links, such as distal outer link 441_{D}, whose perspective view is illustrated in FIG. 20F. In some embodiments, distal outer link 441_{D} can comprise one or more function elements, such as a component selected from the group consisting of: a camera such as camera 448a, one or more light emitting components such as LEDs such as LEDs 448c; an electronics module; an irrigation lumen and/or nozzle such as irrigation port 448b; and combinations of these. In some embodiments, distal outer link 441_{D} can comprise one or more side ports, such as the two side ports 450 shown (e.g. configured to receive a tool support as described herein). In some embodiments, one or more (non-distal) outer links 441 can include one or more similar side ports, not shown but is the same as or similar to side ports 455 described herein with respect to FIGs. 4B and 4C.

The channels (i.e. lumens) 422, 442 and working channel recesses 425, 445 of inner links 421 and/or outer links 441 can comprise an hour-glass or other tapered profiles. For example, the tapered or hour-glass profile 427 of the inner cable channel 422 is shown in FIG. 19D. The cable channels 442 of the outer links 441 of FIGs. 20A-20C, 20E can have a similar profile. Also, a tapered or hour-glass profile 447a of the recess 445 of the outer link 441 can be seen at FIG. 20E. The inner link recesses 425 can have a similar corresponding profile 447b as seen at FIG. 19B. The hour-glass or other tapered profiles can be configured to prevent pinching of one or more tools or filaments passing therethrough. In some embodiments, the surfaces of the profiles 427, 447a, 447b are constructed and arranged to so that a tool or filament passing through the corresponding channel or recess is permitted to pass through the channel or recess with minimal or no longitudinal resistance. For example, the hour-glass profile 427, 447a, 447b of two consecutive inner and outer links can be configured so that tools or filaments can pass through freely without resistance even when the consecutive links are oriented relative to each other at the most extreme articulation angle permitted between them. In some embodiments, recesses 425 (as shown), recesses 445 (as shown), channels 422 (as shown) and/or channels 442 comprise an hour-glass profile. For example, the tapered or hour-glass profile 427 of the inner cable channel 422 is shown in FIG. 19D. The cable channels 442 of the outer links 441 of FIGs. 20A-20C, 20E can have a similar profile. Also, a tapered or hour-glass profile 447a of the recess 445 of the outer link 441 can be seen at FIG. 20E. The inner link recesses 425 can have a similar corresponding profile 447b as seen at FIG. 19B. The hour-glass profile can be used to minimize the maximum diameter of the channel or recess, such as would be necessary if the channel or recess had a single, straight taper. In some embodiments, one or more recesses 425, recesses 445, channels 422 and/or channels 442 comprise a tapered profile such as is described in applicant's co-pending United States Patent Application Serial No. 13/880,525, filed April 19, 2013, the content of which is incorporated herein by reference in its entirety.

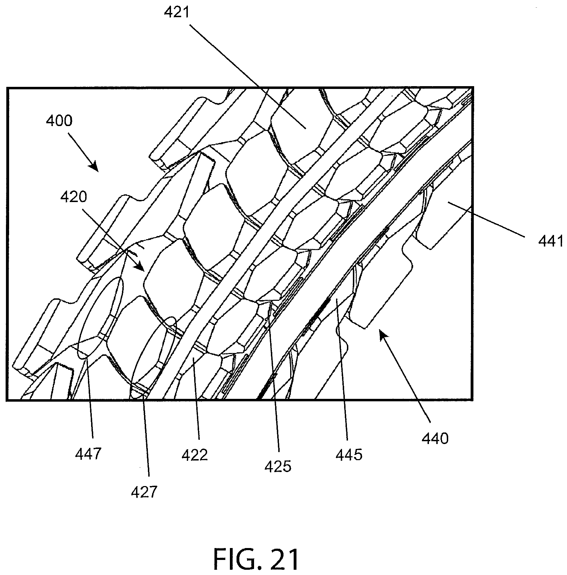

**In** **FIG. 21****,** the hour-glass profiles within articulating probe 400 are illustrated in a side sectional view. Articulating probe 400 comprises inner link mechanism 420 and outer link mechanism 440. Inner links 421 and outer links 441 comprise geometries that define the tapered or hour-glass profiles 427, slots 447 in channels 422 and the working channels created by recesses 425 and 445. In the embodiment of FIG. 21, channels 442 of outer link mechanism 440 comprise a linear tapered profile. In some embodiments, channels 442 of outer link mechanism 440 also comprise an hour-glass profile.

**Referring now to** **FIG. 22****,** a side sectional view of the distal portion of an outer link mechanism is illustrated, consistent with the present inventive concepts. **FIGs. 22A and 22B** illustrate two magnified views of a conical to spherical interface of outer links of FIG. 22, consistent with the present inventive concepts. A distal portion of articulating probe 400 comprises a series of seven outer links 441a through 441g (singly or collectively outer links 441), arranged distally to proximally (i.e., 441a the most distal relative to the other outer links 441b-441 g). Distal link 441a can be constructed and arranged similar to distal outer link 441_{D} described herein at least with reference to FIG. 20F. Articulating probe 400 can be configured such that at least distal outer link 441a and outer link 441b can be steered, while allowing additional adjacent links of outer links 441 to be steered, such as up to the seven outer links 441 shown (e.g. when seven outer links 441 extend beyond the distal end of inner link mechanism 420 and outer link mechanism 440 is steered as described herein). In embodiments, where one outer link, for example, outer link 441g, has a conical distal surface 444g and an adjacent outer link, for example, outer link 441f, has a spherical proximal surface 443f, the contacting surfaces between conical distal surface 444g and the adjacent spherical proximal surface 443f defines a circle, reducing the surface area in each outer link 441 to outer link 441 interface as described herein. In doing so, the spherical proximal surface 443f has less surface area that contacts the linear surface of the tapered or conical distal surface 444g.

In some embodiments, the outer links 441 to be steered are constructed and arranged such that during steering, a series of articulations occur between adjacent outer links 441, for example between outer link 441a and adjacent outer link 441b, between outer link 441b and adjacent outer link 441c, and so on. In doing so, distal outer link 441a begins to articulate prior to next link 441b, which articulates prior to next link 441c and so on. This cascading series of initial articulations can be created in numerous ways, for example, shown in steps 1-6 of FIG. 22C. In some embodiments, a taper angle 0 of each distal surface 444 of outer links 441b through up to 441g (e.g. to allow 7 segment steering) may increase from taper angle Omin (e.g., outer link 441b as shown in FIG. 22B) to θₘₐₓ (e.g., outer link 441g as shown in FIG. 22A), thereby causing an increased mating force (e.g. due to a resultant force vector change) between each set of sequential outer links 441. Since the mating force between outer links 441a and 441b is the smallest, followed by the mating force between outer links 441b and 441c, and so on, articulation during steering initiated with outer link 441a, and sequentially cascades distally. In these embodiments, the taper angle can comprise a set of taper angles selected from any group of increasing angles between 10° and 65°, such as a set of two or more taper angles (e.g. to support steering of two or more outer links 441) increasing from 10° in 1° increments or a set of two or more taper angles increasing from 10° in 5° increments. Alternatively or additionally, other characteristics of outer links 441 can be varied between outer links 441a and 441g, such as a characteristic selected from the group consisting of: other geometric changes such as a geometric change affecting interface force; material change such as a sequential set of lubricity that decreases from outer links 441a to 441g; changes in contacting surface area that cause the desired cascade; and combinations of these.

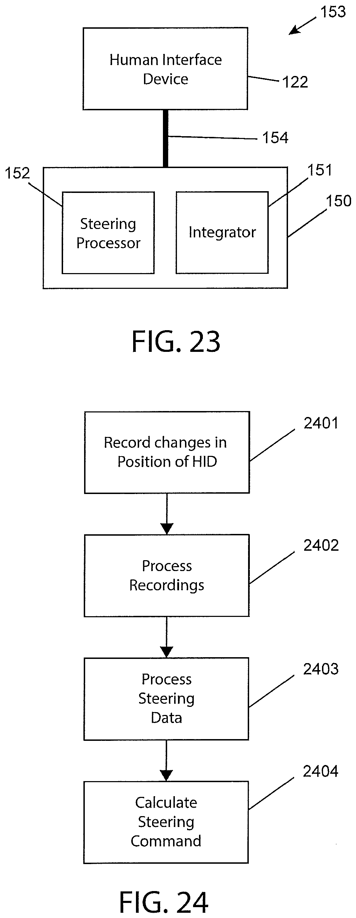

System 100 (e.g. feeder unit 100a and/or interface unit 100b) is constructed and arranged to provide safe and effective operation of articulating probe 400. In some embodiments, system 100 comprises one or more modules described herein in reference to one or more of FIGs. 23 through 28.

In some embodiments, the system 100 includes a processor and a memory for storing and executing some or all of the processes related to the modules described herein. System 100 may take the form of an entirely hardware embodiment, or an embodiment combining software and hardware aspects. Some or all of the processes, can be implemented by computer program instructions, which may be provided to the processor, which may be part of a general purpose computer, special purpose computer, or other programmable data processing apparatus to produce a machine, such that the instructions are stored in the memory, and which execute via the processor of the computer or other programmable data processing apparatus, create means for implementing the functions/acts specified herein.

**FIG. 23** is a block diagram of a steering system 153, in accordance with the present inventive concepts. The steering system 153 includes an HID 122 and a steering module 150. In various embodiments, the steering module 150 can be positioned in one or more of feeder unit 100a and interface unit 100b (generally, 100). Alternatively, steering module 150 can be positioned in a separate or remote hardware unit that communicates with the HID 122 and probe assembly via wired or wireless transmissions known to those of ordinary skill in the art. The steering system 153 communicates with a probe assembly in accordance with some embodiments, for example, feeder 100a of probe system 100 or 400 described herein.

The steering module 150 comprises an integrator 151 and a steering processor 152 for executing some or all processes or computations of a steering procedure, in particular, including the procedure of steps 2401-2403 described herein in connection with reference to FIG. 24.

The HID 122 is constructed and arranged to provide raw steering data to the steering module 150, generated in response to steering motion of the HID manipulated by a surgeon, technician and/or other operator of HID 122. Such an operation is described in detail in United States Patent Application Serial Numbers PCT/US2011/044811, filed July 21, 2011 and PCT/US13/54326, filed August 9, 2013, the content of each of which is incorporated herein by reference in its entirety. For example, the raw steering data 154 can include data related to at least one of the position, movement time, velocity, and/or acceleration of the HID 122, for example as controlled by an operator, which be combined into an output signal or signals, for example, to establish one or more positions of the HID 122.

In normal use, the raw steering data 153 can also include undesirable motion data, such as jitter or the like. Such jitter or shaking of the HID 122 can be imparted on the HID 122 through involuntary movements of the user. For example the user's natural heartbeat or breathing can induce a rhythmic jitter signal into the HID 122 which can manifest itself in the raw steering data produced by the HID 122. Other undesirable motion can occur when an operator makes a sudden or abrupt movement, which can be unintentional.

The a filter or other data processing unit, integrator 151 integrates (or otherwise filters) and processes the received raw steering data 154 to remove certain undesirable motion data included in the raw steering data and to produce a filtered steering signal that is output to the steering processor 152. The steering processor 152 processes the filtered signal received from the integrator 151 to indicate to the probe assembly 400 information about the motion of the HID 122 absent the jitter or undesirable motion otherwise present at the HID 122 during operation.

**FIG. 24** is a flow chart of a steering process, in accordance with the present inventive concepts. In describing the steering process, reference is made to elements of the steering system of FIG. 23. Accordingly, some or all of the steering process can be stored in a memory and executed by a processor of the steering system 153 of FIG. 23. Alternatively, some or all of the steering process can be stored and executed at a remote computer which communicates with the steering system.

In step 2401, a change in position (e.g. a position, time, velocity, acceleration, or other motion-related signal that includes position and time as elements) of the HID 122 is monitored. As described herein, during the normal course of operation, such changes in position are intended by the operator. However, at times, a change in position can occur by jitter or related abrupt, sudden, or other unexpected motion of the HID when the HID is manipulated by the operator. This results in raw steering data that can be output by the HID as a raw data, or recorded by the HID at step 2402, or both. Thus, the raw data can include undesirable motion-related data such as a jitter. The resulting raw data from the HID 122 corresponding to a change in position of the object, e.g., articulating probe 400, manipulated by the HID 122 can be input to the steering processor 152.

In step 2403, the steering data is processed by the processor 152. In some embodiments, the processor performs a mathematical process including integrating the velocity signal measurements that are recorded, which may include the removal of undesirable HID motion from the steering data such as jitter and to produce an integrated steering signal that is output to the steering processor 152. Integrating, or otherwise filtering the steering data can remove signals related to unintended motion of the HID, noise associated with the system, and/or other undesired signals, producing a clean steering signal to be output to the steering processor 152 to produce a smooth robotic motion. The steering processor 152 processes the filtered signal received from the integrator 151 to indicate to the probe assembly 400 information related to the motion of the HID 122 absent the jitter or undesirable motion otherwise present at the HID 122 during operation.

In step 2404, a steering command is calculated based on the analysis of step 2403. The steering command is output to the feeder unit 100a to activate the cable motors, e.g., cable motors 212 shown at FIG. 8 for manipulating the articulating probe 400.

Steering module 150 and/or the method of steps 2401 through 2404 can be configured to improve steering of articulating probe 400, such as to filter or otherwise compensate for tremors or other unintended motion (e.g. unintended reciprocal or small motion of the HID) that may be present when an operator such as a surgeon controls HID 122. During the operation of probe system 100, steering data from HID 122, for example, position, velocity or other movement-related information, is monitored by steering module 150 at a pre-determined rate, such as a rate of between 1Hz and 10,000Hz, such as a rate of approximately 1000Hz. High sampling rates can result in detection of input errors such as those caused by operator tremor, and can correlate to undesired motion of articulating probe 400. The integrator 151 can be used to filter any undesired input signals, for example input signals at an undesired frequency to the controller controlling the movement of the articulating probe 400 in response to the steering commands output from the steering processor 152, to reduce this undesired motion of articulating probe 400 and/or otherwise produce a smooth output. By changing the interval of integration at the steering module 150, the filtering parameters can be changed to allow either more or less of the high frequency input to pass down to the distal tip of probe 400, which is responsive to the movement of the HID 122 according to the steering commands output from the steering module 150.

In some embodiments, a scale factor is applied upon operator input commands received from HID 122. In some embodiments, the scale factor is adjustable, such as adjustable between a range of 0.1 and 1.0. Scale factors can be utilized to modify a sampling rate of the monitoring of the steering commands, and adjust between fine (small scale factor) and coarse (large scale factor) motion control by HID 122.

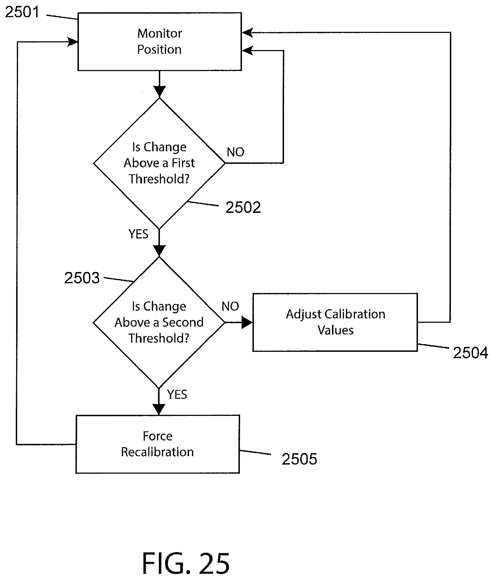

Referring now to FIG. 25, a flow chart of a method for determining the need for a calibration procedure is illustrated, consistent with the present inventive concepts. In describing the method, reference is made to one or more figures herein.

In step 2501, the position of feeder unit 100a is monitored (e.g. a monitoring of a position and/or a change in position), such as with one or more sensors, such as position sensor 225 described herein. The sensor can comprise an accelerometer or other movement sensor used to measure displacement of feeder unit 100a or a sensor configured to measure the position of feeder unit 100a from which displacement of feeder unit 100a can be calculated. The sensor can comprise a gravitational and/or other static position sensor, such as a static position sensor comprising multiple mercury switches or similar switches oriented and arranged to determine the position of an object relative to the force of gravity, e.g., gravitational bias. The static position sensor can be monitored over time such that a displacement of feeder unit 100a can be determined based on a change in the static position. In some embodiments, the sensor provides a signal, which can be used by the system during an operation, for example, to adjust for an "effective weight" of the motor assembly 212 on the load cell 221, more specifically, the weight of the motor assembly 212 absent any extraneous forces on the motor 212 such as tension on the cable about a pulley coupled to and controlled by the motor 212, as described in reference to Fig. 28 herein.

In step 2502, the magnitude of measured displacement (e.g. change in angular orientation) of feeder unit 100a can be compared to a threshold, such as a pre-determined and/or operator settable first threshold. If the measured displacement does not exceed the first threshold, step 2501 can be repeated. If the measured displacement does exceed the first threshold, step 2503 can be performed in which the measured displacement is compared to a second threshold, such as a threshold of greater magnitude than the first threshold. If the measured displacement is less than the second threshold (but greater than the first threshold), step 2504 can be performed in which an adjustment of one or more calibration values is made, such as to adjust the amount of compensation for the effective weight of a motor assembly upon a load cell (e.g. adjusting for the weight of motor and/or motor mount upon a load cell 221 as described hereabove). If the measured displacement is more than the second threshold (as well as the first threshold), step 2505 is performed in which a second calibration procedure, or recalibration, is required, such as a calibration procedure similar to the procedure described herein in reference to FIG. 28. Accordingly, the system may require recalibration if an undesired motion such as a significant movement of the feeder assembly after the initial calibration is determined (e.g. an angular displacement of more than 15°).

In some embodiments, an alarm or alert condition is entered (e.g. and notified to the operator such as via visual and/or audio signal), when the first threshold and/or the second threshold is reached. In some embodiments, the first and/or second threshold correlate to an undesired position of and/or impact to feeder unit 100a, such that feeder unit 100a needs to be repositioned and/or checked for damage prior to normal operation being initiated. In some embodiments, an alarm is generated that indicates that the system enters a forced maintenance state, wherein the system requires maintenance to return to proper functionality and/or deactivate the alarm state.

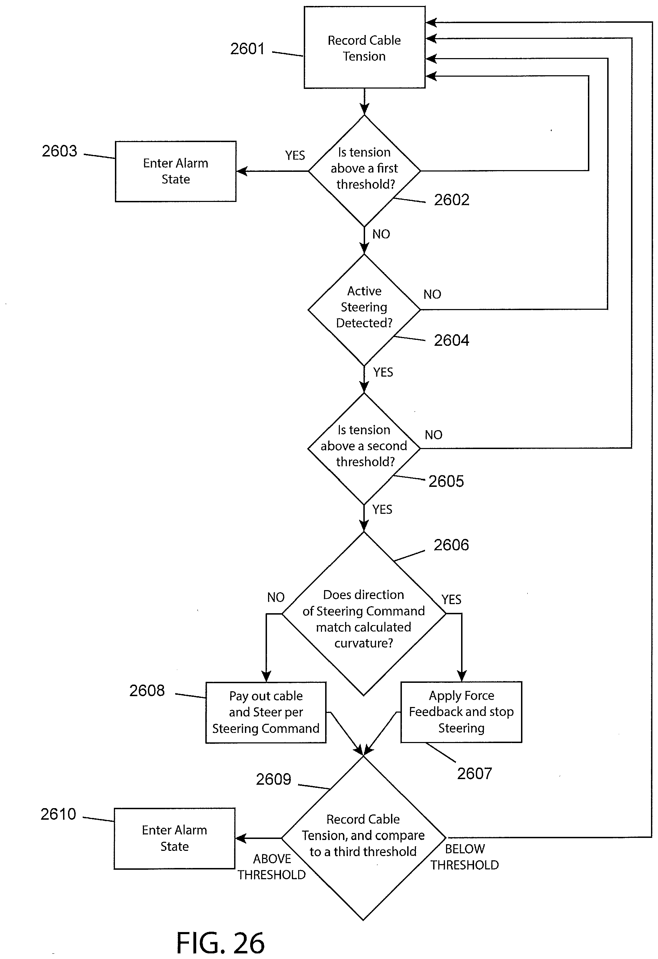

Referring now to FIG. 26, a flow chart of an method for preventing and/or detecting excessive force imparted on the system is illustrated, consistent with the present inventive concepts. In particular, STEPs 2601 through 2610 illustrate a series of steps used to prevent and/or detect undesired force placed and/or otherwise being present on a cable, such as a cable used to steer and/or lock articulating probe 400. Cable tension can be monitored in numerous ways, such as via load cells 221 described herein and/or by monitoring motor current, motor rotation such as via a motor encoder, and the like. In some embodiments, system 100 is configured to prevent the tension in any cable from exceeding approximately 50% of the expected break force of the associated cable. In other embodiments, cable tension is measured at the carriage drive motors 212. Motor current, motor encoder, carriage position (e.g., linear sensor), or the like can alternatively or additionally be monitored in accordance with some or all steps of the following method.

In step 2601, tension or related force data in one or more cables is recorded, such as has been described herein, for example, stored in memory for subsequent retrieval and use by the a computer processor. In step 2602, the cable tension is compared to a first threshold, such as a threshold of at most 50lbs for an inner link mechanism 420 (locking) cable or at most 15lbs for an outer link mechanism 440 (locking and steering) cable. In some embodiments, the threshold can be user defined. If the tension is above the first threshold, step 2603 is performed, in which the system enters an alarm state. An example of an alarm state is in which operation of the articulating probe is stopped, an alert is given to the operator, power to cable motors 212 is removed, and/or tension in one or more cables is reduced. If at step 2602 the tension is not above the first threshold, step 2604 is performed. In some embodiments, the cable tension is compared to the first threshold in hardware circuitry connected to a load cell, such that when the first threshold is identified by the hardware circuitry, a hardware-driven alarm state results in step 2603. In these embodiments, the maximum tension can comprise a threshold of no more than 121bs, 151bs, 181bs, 21lbs or 24lbs (e.g. for a cable 350 of outer link mechanism 440 described herein) or no more than 441bs, 541bs, 641bs, 74lbs or 84lbs (e.g. for a cable 350 of inner link mechanism 420 described herein). Alternatively or additionally, the cable tension is compared to the first threshold implemented at a software program of system 100, which is stored in memory and executed by a computer processor. The system 100 receives a signal from a load cell, such that when the first threshold is identified by the software program, an alarm state results in step 2603, similar or the same as an alarm state described herein. In these embodiments, the maximum tension can comprise a threshold of no more than 9lbs, 121bs, 151bs, 181bs or 21lbs (e.g. for a cable 350 of outer link mechanism 440) or no more than 30lbs, 40lbs, 50lbs, 60lbs or 701bs (e.g. for a cable 350 of inner link mechanism 420).

In step 2604, a determination is made whether the system 100 is in a steering mode, i.e., whether active steering is being performed. In a steering mode, as described herein in reference to the operation of system 100, the probe 140 is articulated by one or more steering cables, which are monitored by sensors as described herein. If steering is not being performed, step 2601 is repeated. If steering is being performed, step 2605 is performed.

In step 2605, the recorded cable tension (of step 2601) is compared to a second threshold, such as a threshold less than the first threshold. In some embodiments, the second threshold comprises a threshold of no more than 3lbs, 5lbs, 7lbs, 9lbs, 11lbs, 13lbs or 151bs. If the recorded tension is not above the second threshold, step 2601 is repeated. If the recorded tension is above the second threshold, step 2606 is performed. In some embodiments, step 2605 is only performed for cables of an outer link mechanism 440, such as when the inner link mechanism is not actively steered by system 100.

In step 2606, the direction of steering (e.g. a steering command entered by an operator into HID 122) is compared to the calculated curvature of articulating probe 400, such as curvature geometry using inverse kinematics (e.g. calculated at each advancement, retraction and/or steering of articulating probe 400 to determine its three dimensional geometric configuration), to determine if the increased cable tension may be caused by the steering command. If the direction of steering matches the calculated curvature of the distal portion of articulating probe 400, (i.e. the system is attempting to steer in the direction the probe is currently curved), step 2607 is performed. If the direction of steering does not match the calculated curvature of the distal portion of articulating probe 400 (i.e. the system attempts to steer opposite the direction the probe 400 is currently curved, for example to straighten the probe 400), step 2608 is performed.

In step 2607, force feedback is presented to the operator (e.g. via a force-feedback based HID 122), and steering is stopped (e.g. all motion of articulating probe 400 is stopped). This force feedback (e.g. pressure or vibration applied to HID 122) can be applied to alert the user that the probe has reached a maximum curvature. Subsequently, step 2609 is performed. Note that the system will remain in a state with the steering stopped until a different steering command from the operator is received.

In step 2608, the cable with the tension above the threshold is advanced, or paid out. The cable being paid out can comprise one or more cables (e.g. of three steering cables) that are not being retracted during the current steering maneuver (e.g. one or more cables that may be transitioning from the inside of a curve to an outside of a curve due to the current steering maneuver). The amount of cable paid out can comprise a length of approximately 2.5mm, 5mm, 10mm, 15mm and/or 20mm. In some embodiments, cable was already being paid out (e.g. automatically, as determined by a steering algorithm and due to the direction of desired steering), and the amount of cable being paid out in step 2608 is in addition to a "standard" amount based on the steering command (i.e. an extra amount delivered to prevent excessive tension in the cable). Subsequently, step 2609 is performed.

In step 2609, cable tension is again recorded and compared to a third threshold. The third threshold can be similar to the second threshold. In some embodiments, the third threshold can be different than the second threshold, such as higher than the first threshold. In some embodiments, the third threshold is similar to the first threshold. If the cable tension is not above the third threshold, a return to step 2601 is performed. If the cable tension is above the third threshold, step 2610 is performed in which the system enters an alarm state, such as a similar or dissimilar alarm state to step 2603 (e.g. an alarm state in which operation of the articulating probe is stopped, an alert is given to the operator, power to cable motors 212 is removed, and/or tension in one or more cables is reduced).

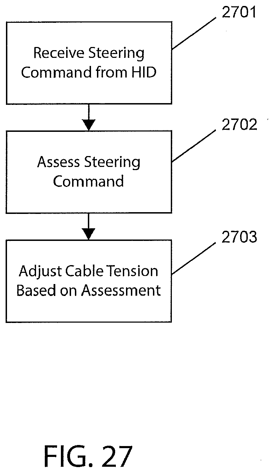

Referring now to FIG. 27, a method for detecting and/or reducing unintended motion of articulating probe 400 is illustrated, consistent with the present inventive concepts. In some embodiments, unintended motion at the distal end of articulating probe 400 is reduced when inner link mechanism 420 and/or outer link mechanism 440 transitions between locked and unlocked states or modes. In these embodiments, the method illustrated in steps 2701 through 2703 described herebelow can be configured to attempt to anticipate an upcoming transition to the locked mode, and confirm and/or cause each of the locking cables to be at a tension level approaching the locked tension level. A transition from a steering mode, also referred to as a flexible mode, to a locked mode can be anticipated when a user input command correlates to a desired rate of motion of probe 400 of less than a threshold (e.g. 5mm/sec). When a user input command correlates to a desired rate of motion higher than the threshold, system 100 can enter a steering state or mode, for example when tension in one or more steering cables is reduced, such as by paying out additional cable (e.g. by paying out 1mm, 2mm, 3mm, 4mm or 5mm of cable), to allow for proper steering performance. When a user input command correlates to a desired rate of motion lower than a threshold (e.g. 5mm/sec), system 100 can enter an "anticipation" mode, for example when tension in on or more steering cables is increased, such as by taking up cable (e.g. by taking up 1mm, 2mm, 3mm, 4mm or 5mm of cable), to pretension cables for locking, while still allowing fine adjustments of probe 400.

In step 2701, a steering command is received from an operator via HID 122. The steering command can be similar or the same as other steering commands described herein. In step 2702, the steering command is assessed to quantify and/or qualify the steering command. In some embodiments, the assessment of step 2702 comprises an assessment of the "aggressiveness" of the steering command, such as an assessment correlating to the velocity and/or acceleration of movement of an operator on an input component of HID 122. An example of aggressive steering of the HID may be a jerking motion or other unintentional motion, in which the velocity and/or acceleration is determined to be higher than a predetermined threshold velocity and/or acceleration deemed to be acceptable or non-aggressive, or otherwise less than the predetermined threshold.

In step 2703, tension within one or more steering cables can be adjusted based on the assessment performed in step 2702. For example, if it is determined that aggressive steering is intentionally being performed, and one or more steering cables need to be paid out (i.e. advanced to allow steering in an opposing direction to the cable being paid out) additional cable may be paid out than if less aggressive steering is detected by the assessment.

The method of FIG. 27 actively manages a cable payout offset that is applied to the two or more (e.g. three) outer mechanism 440 tensioning cables such that 1) when steering "quickly" (as determined by a velocity or acceleration assessment, such as when beginning or in the middle of a steering maneuver, and/or by assessing the amount of steering called for by the user (e.g. the offset of the HID from the neutral position)), the outer links 441 are loosely tensioned with a larger cable payout offset, and 2) when steering "slowly" (e.g. at the end of a steering maneuver, and/or when the offset of the HID from the neutral position is small or minimal), the outer links 441 are more tightly tensioned with a smaller cable payout offset. Thus, the method of FIG. 27 provides for the constant monitoring of the steering input from the user and, in response to steering motion values generated from the monitoring, smoothly varies the tension of one or more cables to anticipate the end of a steering move by tightening the tensioning cables as the steering command slows. Once the steering command ends (i.e. the user is no longer directing the probe to steer via the HID or other input mechanism), articulating probe 400 is already in a partially locked state- because the cables are tensioned thus reducing the additional tension that is required to fully lock articulating probe 400 (e.g. reducing unwanted motion caused by applying tension to cables). The method of FIG. 27 can smoothly ramp cable payout from low to high tension based on the assessment performed in step 2702 (e.g. slower payout when less aggressive steering detected).

**FIG. 28** is a flow chart of a calibration procedure, in accordance with the present inventive concepts. In describing the calibration procedure, reference is made to elements of the probe system 100 of FIGs. 1-22. Accordingly, some or all of the steering process can be stored in a memory and executed by a processor of the probe system 100 of FIGs. 1-22. Alternatively, some or all of the steering process can be stored and executed at a remote computer which communicates with the probe system 100.

In some embodiments, the calibration procedure can be performed based on the current position and angular orientation of the feeder assembly 102 described herein. In particular, calibration and/or re-calibration may be performed to account for variation in forces applied to one or more cables 350 and/or load cell 221 of feeder assembly 102, as a result of the change in position of feeder assembly 102. For example, probe system 100 can execute one or more calibration procedures to calibrate one or more load cells, for example, a load cell 221 of FIG. 8A, which is used to measure tension in a locking and/or steering cable of the present inventive concepts, such as when the load cell 221 is engaged with a motor assembly rotatably attached to a base assembly 200 and configured to drive a bobbin containing the cable, the bobbin in turn engaging capstan 216, as described herein.

The calibration procedure, when performed on the load cell 221, can compensate for changes in position of the feeder assembly 102, resulting in changes in gravitational forces applied to the load cell 221, as well forces applied to load cell 221 via attached structures caused by gravity or other environmental sources. The calibration procedure of steps 2801 through 2805 can be performed multiple times, on different load cells, such that different calibration parameters can be generated for each. Multiple calibration procedures can be performed simultaneously or sequentially.

The rotational force applied by the motor assembly, such as a motor assembly comprising motor 212 and/or motor mount 218 described herein, to the load cell 221 correlates to tension in the cable. In these and other configurations, the load cell 221 may also measure one or more undesired loads (e.g. not desired for cable tension measurement) that is not related to cable tension, such as a load due to a force applied by the weight (e.g. due to gravity) of the motor assembly. At the motor assembly, a weight-driven load on the load cell may be variable, based on the relationship of the motor assembly to the force of gravity. Accordingly, the calibration procedure of FIG. 28 can be performed to determine the specific load due to the weight of the motor assembly that is present at the time of use, e.g. based on the geometric position of the motor assembly relative to the force of gravity.

In step 2801, prior to the start of load cell calibration, a determination is made whether the calibration of one or more load cells 221 is required, for example, when feeder assembly 102 has undergone a change in orientation such that would change the direction of the gravitational forces applied to the load cell 221.

Load cell calibration can be 333initiated at step 2801a in response to an event such as a determination that use of a feeder assembly is about to occur and calibration has not yet been performed or a system start or restart has occurred; top assembly 300 is seated and locked into position on base assembly 200 (see FIG. 11), a detection of change in position of the feeder assembly 102, a calibration has been performed but the feeder assembly has subsequently been reoriented (e.g. as detected by a position sensor such as sensor 225 described herein), an undesired state has been detected by the system, a calibration is requested by an operator, or combinations of these.

In step 2802, the motor assembly 212 may be driven to cause rotation of a cable pulley, for example, at bobbin 316a, such that cable, for example, steering cable 350 of FIG. 5B extending between cable bobbin 316a and links at articulating probe 400, is advanced a preset length, such as to slacken ("pay out"), causing a condition in which little or no force is applied to the load cell 221 due to cable tension.

In step 2803, the feeder assembly 102 and/or motor assembly orientation can be performed, such as by receiving and processing a signal provided by position sensor 225 that detects feeder assembly 102 position or orientation, or both. The orientation data can be used to calculate the expected gravitational forces applied to load cell 221 such that the calibration can account for the gravitational forces. This orientation data can be recorded (e.g. stored in electronic memory), and retrieved by a processor for use in future comparisons and/or for use in one or more processes, for example, software programs stored in memory and executed by a processor, that compensate for and/or otherwise use the orientation information. This orientation data can include but not be limited to yaw, pitch and/or roll of the base assembly 200, or data corresponding to any number of degrees of freedom of the base assembly 200. The orientation of the base assembly 200 can be determined by estimating one or more of the degrees of freedom, for example, yaw, pitch, roll of the base assembly 200 with the position sensor.

In step 2804, zero-tension data from the load cell is recorded (e.g., stored in electronic memory) (e.g. a number of samples). The zero-tension data can comprise a set of data that is averaged or otherwise mathematically processed.

This zero-tension data can correlate to a correction factor (e.g. offset) used to determine cable tension. The zero-tension data can correlate to a load applied to the load cell due to the weight of the motor assembly, since cable tension is currently at or near zero. For example, in embodiments including a plurality of load cells, a number of samples in the load cell sensors are recorded and averaged to determine an offset value. While the base assembly 200 is at a particular orientation (see step 2803), the offset value is provided for adjusting for the gravitational bias at that particular orientation. For example, based on the orientation of base assembly 200, trigonometric techniques can be employed to determine the impact of the weight of base assembly 200 on one or more load cells. The offset value/ zero-tension data can be used to produce a more accurate load cell measurement of the cable tension during use of the system.

Accordingly, in step 2805, operation of the probe assembly is initiated, including steering, advancement, retraction, locking and un-locking of the articulating probe 400, based on the measured cable tension compensated for any or all undesired loads on the one or more load cells 221, as described herein. In some embodiments, the tension in each cable is brought to a predetermined value prior to any advancement or steering maneuver, such as a tension of 1N, 3N, 5N, 7N or 10N. In some embodiments, the amount of tension in one or more cables (e.g. each steering and/or locking cable) is kept above a minimum force, such as a minimum force above 1N, 3N, 5N, 7N or ION. Maintenance of the minimum force can be configured to prevent any undesired hysteresis effects or other undesired effect, such that might otherwise be encountered as the force on the load cell transitions around zero force.

The calibration procedure of steps 2801 through 2805 can be performed on multiple cable-driving motor assemblies, simultaneously or sequentially, such as the four motor assemblies described herein. Alternatively or additionally, a calibration procedure is performed on one or more carriage assembly driving motor assemblies. In other embodiments, the calibration procedure can optionally be initiated in response to a restart of the system, in response to a latching of the feeder unit, or when the orientation of the base changes significantly, as determined by the position sensor.

While the preferred embodiments of the devices have been described in reference to the environment in which they were developed, they are merely illustrative of the principles of the present inventive concepts. Modification or combinations of the above-described assemblies, other embodiments, configurations, and methods for carrying out the invention, and variations of aspects of the invention that are obvious to those of skill in the art are intended to be within the scope of the claims. In addition, where this application has listed the steps of a method or procedure, which does not form part of the scope of the invention, in a specific order, it may be possible, or even expedient in certain circumstances, to change the order in which some steps are performed, and it is intended that the particular steps of the method or procedure claim set forth herebelow not be construed as being order-specific unless such order specificity is expressly stated in the claim.

## Claims

1. An articulating probe system (100a) comprising:
an articulating probe (400) constructed and arranged to articulate in at least one degree of motion and to transition between a flexible state and a rigid state, the articulating probe comprising a plurality of links (440) between a proximal link and a distal link;
a feeder assembly (300) in communication with the articulating probe to apply forces on the articulating probe to cause the probe to articulate and to transition between the flexible state and the rigid state; and
a plurality of cables (350) in communication with the plurality of links;
the feeder assembly further comprising:
a plurality of cable bobbins (316a) at which proximal portions of the plurality of cables are wound; and
drive assemblies (320), each corresponding to one of the cable bobbins, for driving the cable bobbins, **characterised by** the drive assemblies including motion resistance mechanisms (377) that substantially prevent rotation of the cable bobbins as a result of forces transferred through the cables, as encountered by the articulating probe.

2. The articulating probe system of claim 1, wherein the drive assemblies each comprise:
a motor;
a gear assembly that is driven by the motor; and
a capstan that is constructed and arranged to be driven by the gear assembly and to communicate with the cable bobbin.

3. The articulating probe system of claim 2, wherein
the gear assembly comprises a worm gear assembly, and/or
the gear assembly comprises at least one of a ratchet and pawl mechanism or a magnetic position holding assembly.

4. The articulating probe system of claim 2 wherein the motor comprises at least one of a stepper motor, a closed-loop servomotor, or a DC motor having a shorted drive inductor.

## Patentansprüche

1. Ein Sondensystem mit Gelenken (100a) welches Folgendes umfasst:
eine Sonde mit Gelenken (400), welche so ausgebildet und angeordnet ist, um sich zumindest in einem Bewegungsgrad zu bewegen und um zwischen einem flexiblen und einem festen Zustand zu wechseln, wobei die Sonde mit Gelenken eine Vielzahl von Verbindungen (440) zwischen einer proximalen Verbindung und einer distalen Verbindung aufweist;
eine Zuführanordnung (300), welche in Kommunikation mit der Gelenksonde steht, um Kräfte auf die Gelenksonde auszuüben, damit die Sonde veranlasst wird, sich zu bewegen und zwischen dem flexiblen Status und dem festen Status zu wechseln; und
eine Vielzahl an Kabeln (350), die in Kommunikation mit der Vielzahl von Verbindungen stehen;
wobei die Zuführanordnung des Weiteren Folgendes umfasst:
eine Vielzahl von Kabelspulen (316a), auf welche die proximalen Teile der Vielzahl von Kabeln gewickelt sind; und
Antriebsanordnungen (320), welche jeweils passend zu einer der Kabelspulen sind, zum Antreiben der Kabelspulen, **dadurch gekennzeichnet, dass**
die Antriebsanordnungen Bewegungswiderstandsmechanismen (377) beinhalten, welche im Wesentlichen die Drehung der Kabelspulen verhindern, welche aufgrund von Kräften auftritt, die bei Kontakt mit der Gelenksonde durch die Kabel übertragen werden.

2. Das Sondensystem mit Gelenken nach Anspruch 1, wobei die Antriebsanordnungen jeweils Folgendes umfassen:
einen Motor;
eine Getriebeanordnung, welche von dem Motor angetrieben wird; und
eine Antriebsrolle, welche so ausgebildet und angeordnet ist, um von der Getriebeanordnung angetrieben zu werden und mit der Kabelspule zu kommunizieren.

3. Das Sondensystem mit Gelenken nach Anspruch 2, wobei die Getriebeanordnung eine Schneckengetriebeanordnung umfasst, und/oder die Getriebeanordnung zumindest einen Sperrklinkenmechanismus oder eine magnetische Positionshaltungsanordnung umfasst.

4. Das Sondensystem mit Gelenken nach Anspruch 2, wobei der Motor zumindest einen Schrittmotor, einen closed-loop Servomotor oder einen Gleichstrommotor mit einem gekürzten Antriebinduktor aufweist.

## Revendications

1. Système de sonde articulés (100a) comprenant :
une sonde articulée (400) construite et agencée pour s'articuler dans au moins une amplitude de mouvement et pour faire la transition entre un état flexible et un état rigide, la sonde articulée comprenant une pluralité de liaisons (440) entre une liaison proximale et une liaison distale ;
un dispositif d'alimentation (300) en communication avec la sonde articulée pour appliquer des forces sur la sonde articulée afin de provoquer l'articulation et la transition de la sonde entre l'état flexible et l'état rigide ; et
une pluralité de câbles (350) en communication avec la pluralité de de liaisons ;
le dispositif d'alimentation comprenant en outre :
une pluralité de bobines (316a) de câble au niveau desquelles des parties proximales de la pluralité de câbles sont enroulées; et
des dispositifs d'entraînement (320) correspondant chacun à l'une des bobines de câble, pour entraîner les bobines de câble, **caractérisé en ce que**
les dispositifs d'entraînement comprennent des mécanismes de résistance au mouvement (377) qui empêchent sensiblement la rotation des bobines de câble en raison des forces transférées à travers les câbles, telles que rencontrées par la sonde articulée.

2. Système de sonde articulée selon la revendication 1, dans lequel les dispositifs d'entraînement comprennent chacun:
un moteur ;
un ensemble d'engrenages entraîné par le moteur ; et
un cabestan qui est construit et agencé pour être entraîné par l'ensemble d'engrenages et pour communiquer avec la bobine de câble.

3. Système de sonde articulée selon la revendication 2, dans lequel l'ensemble d'engrenages comprend un ensemble d'engrenages à vis sans fin, et/ou l'ensemble d'engrenages comprend au moins un mécanisme à rochet et à cliquet ou un dispositif de maintien de position magnétique.

4. Système de sonde articulée selon la revendication 2, dans lequel le moteur comprend au moins un moteur pas-à-pas, un servomoteur en boucle fermée ou un moteur à courant continu ayant une inductance d'entraînement court-circuitée.
